# EUROPÄISCHE PATENTANMELDUNG

(11) **EP 1 550 715 A1**
(43) Veröffentlichungstag der Anmeldung: **06.07.2005**
(21) Anmeldenummer: 03029961.4
(22) Anmeldetag: 30.12.2003
(51) Int. Cl.: C12N 5/00, A61K 38/18, A61K 38/27

(54) **Verfahren zur Regenation von Gewebe**

(71) Anmelder: Bionethos Holding Gmbh, 31275 Immensen (DE)
(72) Erfinder: Bader, Augustines, Dr., 31275 Immensen (DE)
(74) Vertreter: Von Renesse, Dorothea, Dr.

(57) **Zusammenfassung**

Die Erfindung betrifft die Verwendung der hämatopoietischen Wachstumsfaktoren Erythropoietin (EPO) oder Thrombopoietin (TPO), sowie deren Derivate, Analoga oder Teile zur Förderung der strukturellen Regeneration von Gewebe.

## Beschreibung

Die vorliegende Erfindung betrifft ein Verfahren zur Induktion des strukturellen Wachstums von Gewebe, insbesondere bei der Leberregeneration.

In der Ontogenese werden Wachstumsfaktoren exprimiert, die grundlegende strukturelle und hinsichtlich der Zellzahl numerische Prozesse für den Aufbau eines Gewebes auslösen können. Im wachsenden und im adulten Organismus geht die Fähigkeit, strukturell und funktionell intakte Gewebe aufzubauen oder - im Falle von Gewebeschädigungen - zu regenerieren, allerdings weitgehend verloren. Es wird vermutet, dass die verminderte Expression von Wachstumsfaktoren, die ihrerseits die Expression von für den Gewebeaufbau erforderlichen Proteinen kontrollieren, für diese Reduktion an Regenerationsfähigkeit verantwortlich ist.

Es ist jedoch zumindest von einige Organen bekannt, dass sie selbst im adulten Organismus eine Fähigkeit zur Selbst-Regeneration behalten, die durch Verletzungsprozesse induziert werden kann. So ist beispielsweise die Regenerationskapazität der Leber bereits seit der Antike bekannt. Fast alle anderen Organe können strukturelle Defekte nicht von sich aus entsprechend überbrücken um, das originale Gewebe wiederherzustellen.

Die Leber befindet sich im adulten Organismus in der Regel im ruhenden, das heißt nicht-proliferierenden Zustand, in dem das Organ eine komplexe Vielfalt verschiedener metabolischer Funktionen erfüllen muß. *In vivo* wird die Leber allerdings durch den Verlust von Zellmasse ― z.B. durch eine Leberzellschädigung oder durch einen chirurgischen Eingriff ― erneut zum Wachstum angeregt.

Proliferierendes Lebergewebe ersetzt die funktionellen und anatomischen Strukturen des Organs meist jedoch nicht in der gewünschten Weise, sondern führt in der Regel zu einer Vergrößerung und Hypertrophie des verbliebenen Lebergewebes, bis die ursprüngliche Leberzellmasse ersetzt ist. Die Intensität der Wachstumsantwort ist von dem Ausmaß des Gewebeverlustes abhängig. Der zeitliche Verlauf der Leberregeneration korreliert dabei umgekehrt proportional, das heißt kleine Leberzellverluste werden langsam ersetzt, große Leberzellverluste wesentlich rascher.

Der Ersatz der Organmasse durch Zellproliferation allein stellt daher keine ausreichende Basis für die Therapie eines Patienten mit erheblichem Organschaden dar. Es sind daher verschiedene Ansätze für die Induktion strukturellen Wachstums ― d.h. für ein formschaffendes Wachstum - von Geweben bekannt, die jedoch allesamt noch nicht zu einem befriedigenden Erfolg geführt haben. Ein solches strukturelles Wachstum von Zellen wäre jedoch gerade für therapeutische oder biotechnologische Verfahren von erheblicher Bedeutung.

In der Vergangenheit wurde versucht, Wachstum von Zellen über die Gabe von Wachstumsfaktoren, wie z.B. "Epidermal Growth Factor" (EGF), "Vascular Endothelial Growth Factor" (VEGF) oder "Hepatocyte Growth Factor" (HGF) zu induzieren. Die Wirkung dieser Faktoren auf die Vermehrung primärer Zellen *in vitro* ist jedoch begrenzt. Ihr Einsatz *in vivo* ist dagegen aufgrund ihrer möglichen Nebenwirkungen - z.B. der Aktivierung von Onkogenen ― nicht unproblematisch.

Ein anderer Ansatz basiert auf der Verwendung komplexer heterologer Gewebeextrakte, z.B. aus der Hypophyse oder dem Hypothalamus, zur Induktion der Zellvermehrung beispielsweise von kultivierten Hepatozyten (siehe z.B. US 6,008,047). Die Verwendung tierischer oder humaner Gewebeextrakte ist jedoch vor dem Hintergrund übertragbarer viraler Erkrankungen, wie z.B. BSE, Schweine- oder Schaf-Viren, im Laborbetrieb oder in der klinischen Anwendung problematisch und dokumentiert eher das mangelnde Wissen um die Prozesse, die an dem Aufbau komplexer Organstrukturen beteiligt sind sowie um die eigentlich relevanten Faktoren und deren Einsatz- und Wirkpotentiale. Zudem sind die Extrakte in ihrer Qualität schwer definierbar, da diese u.a. von der Quelle und deren Kultivierungsbedingungen abhängt.

Selbst die wenigen Erkenntnisse aus den klassischen Anwendungen der primären Gewebekulturen lassen sich allerdings nicht unmittelbar auf die Fragestellungen des *tissue engineering* übertragen. So geht das *tissue engineering* in der Regel idealerweise von patientenspezifischen, adulten Zellsystemen aus, die bereits weiter differenziert sind als fötale oder embryonale Zellen. Außerdem handelt es sich bei dem *tissue engineering* sowohl *in situ* als auch *in vitro* um Kokultursituationen, die in der klassischen Anwendung nicht berücksichtigt werden. Es wird dagegen viel eher sogar versucht, Kokulturen aus Endothelzellen, Makrophagen und Fibroblasten, wie sie in der Leber vorkommen, bei der Expansion der parenchymatösen Leberzellen zu vermeiden, da sie nicht erwünscht sind.

Aus der WO 02/092013 A2 ist es bekannt, einem Patienten zur Behandlung von Leberschäden eine therapeutisch effektive Menge von Wachstumshormon (Growth Hormone, GH) zu verabreichen, um damit das natürliche Regenerationsvermögen der Leber zu fördern. Danach hat GH die Wirkung, die Expression des Wachstumsfaktors Fox M1B bei Hepatozyten zur beschleunigten und so das Leberwachstum erneut zu initiieren.

Das Wachstumshormon hat jedoch eine sehr breite und daher unspezifische Wirkung auf das Wachstum von Geweben. Daher werden durch GH-Gabe auch ungewollte Nebenwirkungen oder Überreaktionen z.B. in Form der sogenannten Akromegalie, d. h. einer überschiessenden Verknöcherung mit pathologischen Knochenzuständen, erzeugt. Des weiteren wurde in der Zischenzeit bekannt, dass Fox1M in Basalzellkarzinomen hochreguliert wird. Die Fox-Proteine spielen eine wichtige Rolle in der Regulation der Wachstumsgene bei der Vermehrung, Differenzierung und der Transformation, u.a. auch bei der Aktivierung von sogenannten SONIC HEDGEHOG (Shh) Signalwegen. Diese wiederum sind involviert in der Aktivierung von Basalzellkarzinomen in menschlicher Haut. So konnten Teh et al. zeigen (Cancer Research 2002, August 15; 62-(16): 4773-80), dass die Hochregulierung von FoxM1 in Basalzellkarzinomen eine der wesentlichen Initiations-Mechanismen ist, wodurch die SONIC HEDGEHOG-Signalwege mitogene Effekte in den basalen Kerotinozyten ausüben, wodurch es zu der Entwicklung des weit verbreiteten menschlichen Krebsgeschwürs kommt. Dieses tumorigene Potential von Fox1M Agonisten und die mangelnde Spezifizität und ubiquitäre Präsenz in allen Geweben steht daher der Verarbreichung von GH zur Förderung der Leberregeneration entgegen.

Aufgabe der vorliegenden Erfindung ist es daher, ein Verfahren bereitzustellen, das die Induktion eines im wesentlichen strukturellen Wachstums eines Gewebes induziert. Dieses Wachstum sollte vorzugsweise zu einer im wesentlichen funktionellen und strukturellen Funktionsfähigkeit des betroffenen Gewebes führen.

Erfindungsgemäß wird diese Aufgabe gelöst durch den Einsatz von hämatopoietischen Cytokinen, deren Derivate oder Analoga bzw. Teilen für die strukturelle und funktionelle Leberregeneration. In einer besonders bevorzugten Ausführungsform wird Erythropoietin (EPO) oder ein Derivat, Teilen oder Analogon davon verabreicht. Der erfindungsgemäße Effekt auf das betroffene Gewebe kann jedoch auch durch die Applikation von Thrombopoietin oder Teilen davon erreicht werden.

Es hat sich überraschenderweise gezeigt, dass durch die Applikation von hämatopoietischen Wachstumsfaktoren wie EPO und TPO nicht nur eine Vermehrung der Zellen, sondern auch ein strukturelles Wachstum einsetzt. Dieses Wachstum setzt insbesondere an zuvor traumatisierten Geweben ein. Das dadurch induzierte Wachstum führt *in vivo* zu einer Gewebe-Regeneration im eigentlichen Sinne, d.h. es findet nicht nur proliferatives Wachstum, sondern gerichtetes, differenziertes Wachstum zum Aufbau komplexer Strukturen statt.

Im Kern basiert die erfindungsgemäße Verwendung der hämatopoietischen Faktoren für die Geweberegeneration im wesentlichen auf zwei bisher nicht bekannten Wirkungen des EPO, nämlich einerseits auf der Stimulation des strukturellen Wachstums in synchroner und koordinierter Form von verschiedener Zelltypen untereinander und miteinander (wie z.B. Fibroblasten, glatten Muskelzellen mit Endothelzellen im Gefäßbereich in Verbindung mit einer Neubildung der Architektur eines kompletten Gefäßes unter Berücksichtigung der extrazellulären Matrix (Kollagen, Elastin, Fibronektin, Entaktin)) und einer Komplettierung des eigentlichen parenchymatösen Gewebeverbandes. Dies beinhaltet z.B. die Ausbildung von Hepatozyten mit den verbundenen Kupffer Zellen, Pit- Ito- und Endothelzellen (sog. nicht parenchymale Zellen der Leber). Neben der Ausbildung eines tatsächlichen Gefäßbaumes und dessen Interkonnektion wird somit erfindungsgemäß eine Geweberegeneration im Sinne der *restitutio ad integrum* induziert.

In der Leber führt dies zu einer Mischung aus sinusoidalen Kapillaren im Endstrombereich und vaskulären zu- und abführenden Gefäßen neben dem eigentlichen Parenchymverband der Hepatozyten in einer geordneten 3-D. Struktur.

Wie bereits ausgeführt, setzt die erfindungsgemäße Wirkung der hämatopoietischen Wachstumsfaktoren insbesondere bei traumatisierten Geweben und Zellen ein. Dabei wird der Begriff des Traumas als Gegensatz zu dem Prozess der Histogenese (Gewebebildung) definiert. Es handelt sich demnach bei einem Trauma um einen Prozess, der der Histogenese als Gewebildungsprozess im Individualorgismus an den betreffenden Lokalisationen entgegenwirkt bzw. das Ergebnis der Histogenese zunichte macht. Das Trauma als Gewebeschädigung kann durch eine Vielzahl von Ereignissen ausgelöst werden, z.B. durch Verletzungen, Entzündungen oder durch Autoimmunerkrankungen mit Selbstbeschädigung). Diese Gewebeschädigungen oder -zerstörungen lösen wiederum eine Vielzahl von Reaktionen aus, so z.B. die Aktivierung von Makrophagen, Mastzellen und immunkompetenten Zellen, die chemotaktische, vasoaktive und wundheilungsfördemde Faktoren sezernieren, und dadurch systemische und regioselektive Mechanismen regulieren.

Die Vorteile der erfindungsgemäßen Verwendung der hämatopoietischen Wachstumsfaktoren, insbesondere des EPO, erstrecken sich auf die Regeneration von Geweben aller vier Grundgewebearten, nämlich des Bindegewebes, des Muskelgewebes, des Epithelgewebes und des Nervengewebes. Diese Gewebe leiten sich ontogenetisch entweder aus dem Mesoderm (Bindegewebe, Muskel, Endothel (als besondere Form des Epithels)), dem Endoderm (das den Gastrointestinaltrakt auskleidende Epithel) oder dem Ektoderm (Nervengewebe) ab. In der Vergangenheit wurde gezeigt, dass der EPO-Rezeptor sowohl auf Zellen meso- als auch endodermalen Ursprungs sowie auf neuronalen Zellen exprimiert wird.

In diesen Geweben führt die erfindungsgemäße Verwendung von EPO oder TPO zu einem ortsständigen Recruitment der gewebespezifischen Progenitorenpopulation (Stammzellen), der Migration der Zellen und der Differenzierung bzw. Transdifferenzierung der Zellen in Parenchym- und Strukturzellen. Während und vor dieser Gewebsbildung vermehren sich die Zellen durch die EPO Gabe.

Bei der Anwendung des erfindungsgemäßen Verfahrens beispielsweise für die Leberregeneration kann eine erneute Vervollständigung des zuvor geschädigten Organs zu einem kompletten parenchymatösen Gewebeverband erreicht werden, einschließlich der Ausbildung von Hepatozyten mit Kupffer-Zellen, Pit-, Ito- und Endothelzellen. Mit der weiterführenden Ausbildung eines Gefäßbaums ist somit erfindungsgemäß die Geweberegeneration als *restitutio ad integrum* möglich.

Ein besonderer Vorteil des erfindungsgemäßen Verfahrens liegt somit darin, daß nicht nur die Mikokapillarisation des regenerierenden Gewebes über eine Endothelaussprossung stimuliert wird, sondern auch die Parenchymregeneration und die Ausbildung der Wandstrukturen gefördert werden. Erst dies führt zu dem gewünschten Ergebnis des koordinierten dreidimensionalen Wachstums zum Aufbau eines funktionsfähigen Organs.

Somit basiert die erfindungsgemäße Verwendung auf einer EPO-Wirkung, die weit über die bisher bekannte EPO-Wirkung als angiogentischer Faktor auf die Endothelzellenvermehrung hinausgeht (Journal of Nephrology 2002 15, 97 bis 103). Da mikrovaskuläre Strukturen wie Kapillaren und Sinusoide lediglich aus Endothelzellenauskleidung bestehen und keine eigene Wandstruktur aufweisen, konnte ausgehend von der angiogenetischen Wirkung des EPO bisher jedoch lediglich darüber spekutiert werden, ob EPO auch bei der Gefäßneubildung und Wundheilung eine gewisse Bedeutung zukommen könnte (Journal of Nephrology 2002 15,97 bis 103). Eine Substantiierung dieser Spekulationen blieb bislang aber aus.

Daher ist es vorliegend umso bedeutender, dass erstmals der Nachweis der Wirkung des EPO auf das synchronisierte und koordinierte Wachstum der Gefäße selbst, d.h. einschließlich der Ausbildung der Wandstrukturen und die Parenchymregeneration gelungen ist.

Ein weiterer Vorteil des erfindungsgemäßen Verfahrens besteht darin, dass das strukturelle Wachstum nicht notwendigerweise eine vorgegebene organische oder anorganische dreidimensionale Struktur als Ausgangspunkt benötigt, sondern viel eher eine Organ(teil)struktur *de novo* schafft. Somit kann die Applikation der hämatopoietischen Wachstumsfaktoren eine deutlich beschleunigte Selbst-Regeneration eines beschädigten Gewebes induzieren, was in der klinisch-therapeutischen Anwendung der Erfindung von großer Bedeutung ist.

In einer besonders vorteilhaften Ausführungsform der Erfindung werden die hämtopoietischen Wachstumsfaktoren eingesetzt, um die Regeneration eines Gewebes zu induzieren, das traumatisch geschädigte Bereiche aufweist. In diesen Gewebeabschnitten kann damit nicht nur ein Verschluß der Wunde durch die Ausbildung eines Granulationsgewebes mit einsetzender Angiogenese angeregt, sondern die Neubildung der gewebespezifischen dreidimensionalen Struktur aus extrazellulärer Matrix z.B. aus Kollagen, Elastin, Fibronektin oder Etnaktin.

Erfindungsgemäß werden hämatopoietische Wachstumsfaktoren eingesetzt. Dabei handelt es sich vor allem um Thrombopoietin, Erythropoietin und das Wachstumshormon (GH) sowie deren funktionell und strukturell homologe Analoga, Derivate und /oder Teilen davon. Insbesondere aber werden EPO oder TPO bzw. deren Teile, Derivate oder Analoga eingesetzt. Es eignen sich auch deren mimetische Peptide (siehe unten). Zu den hämatopoetischen Wachstumsfaktoren zählen weiterhin G-CSF und GM-CSF.

Bei den hämatopoietischen Wachstumsfaktoren handelt es sich um linksseitige Vierfachhelixbündelproteine mit einer Orientierung rauf - rauf -- runter - runter mit zwei überreichenden Schleifen, die die ersten beiden und die letzten beiden Helixstrukturen miteinander verbinden (Livnah O. et al., Science 1999, 283 (5404): 987-90 und Ultsch M.H. et al, Blood 1995, 86 (2): 540-7. Die jeweiligen RBD Domainen (rezeptorbindenden Domänen) besitzten eine ausgeprägte Homologie mit EPO. Thrombopoietin, Erythropoietin und Wachstumshormon binden an den MPL-Rezeptorkomplex. In der Publikation von Youssoufianh et al. in Blood 1993, 819 2223 bis 36, Structure function and activation of the Erythropoietin receptor) wird eine produktive Dimerisation beschrieben. Erythropoietin und Thrombopoietin, mimetische Peptide (EMP und DMP) und weiterhin nicht-peptidische kleine Moleküle sind ebenso funktionell, wenn auch auf einer niedrigeren molaren Basis (Publikationen: Wrighton NC et al. Small peptides as mimetics of the protein hormone erythropoietin Science 1996, 273 (5274) 458-64 und Cwirla S. E. et al. Peptide agonist of the thrombopoeitin receptor as potent as the natural cytokine, Science 1997, 27653191696-9 und Qureshi S.A. et al. Mimicry of Erythropoietin by non peptid molecules, Proceedings National Academy of Sciences PNAS USA 1999, (9621): 12156-61). Die Bindung von EMP 1 an den Rezeptor geschieht an Bindungsstellen, die homolog zu den Hotspots der Wachstumshormonrezeptorverbindungen sind. Die Struktursequenzen von Thrombopoietin sind im Detail in EP 1 201 246 A2 beschrieben. Die Struktursequenzen von Erythropoietin sind im Detail in der europäischen Patentanmeldung EP 84 308 654.7 beschrieben.

In einer besonders vorteilhaften Ausführungsform der Erfindung wird EPO verwendet, das *in vivo* die Bildung von Erythrozyten sowie die Zellteilung und Differenzierung von Erythrozyten-Vorläuferzellen stimuliert. EPO ist entweder aus Urin isolierbar oder aber rekombinant herstellbar. Die Herstellung eines rekombinanten humanen EPO ist Gegenstand der WO 86/03520. Des weiteren ist EPO Gegenstand der EP 0 148 605, EP 0 205 564, EP 0 209 539, EP 0 267 678 oder EP 0 411 678.

Es können jedoch auch Derivate des nativen oder rekombinanten EPO eingesetzt werden. So ist beispielsweise ein EPO-Derivat bekannt (EP 0 640 619 B1), das zusätzliche Glykosylierungsstellen und somit einen höheren Kohlenhydratanteil mit 22 Sialsäureresten aufweist. Der Vorteil dieser Modifikation besteht darin, daß mit einem erhöhten Anteil an Sisalsäure die Halbwertzeit des EPO im Plasma zunimmt, da nur ein nicht-sialisiertes EPO an dem am EPO-Abbau beteiligten Galactose-Rezeptor der Leber binden kann. Dieses EPO-Derivat ― bekannt unter der Abkürzung NESP (*Novel Erythropoiesis Stimulating Protein* oder Darbepoetin) ― weist zwar im Vergleich zum rekombinanten EPO eine an fünf Positionen veränderte Aminosäuresequenz auf. Es entspricht aber in seiner Wirkung auf die Stimulation der Erythrozytenbildung im wesentlichen dem nativen oder rekombinanten EPO (Fisher, J.W.: Erythropoietin: Physiology and Pharmacology Update. Erythropoietin 2003, 1-14). Die EP 0 640 619 B1 wird hinsichtlich der Struktur und Herstellung des NESP vollumfänglich in Bezug genommen.

Das NESP kann zur weiteren Verlängerung der *in vivo* Halbwertzeit vorteilhafterweise noch mit Polyethylenglykol (PEG) chemisch konjugiert werden, ohne daß damit eine Veränderung der biologischen Aktivität verbunden wäre. Ein derart modifiziertes NESP ist aus der WO 01/76640 bekannt, die hinsichtlich der Struktur und Bereitstellung dieses EPO-Derivats vollumfänglich in Bezug genommen wird.

Aus der WO 02/49673 A2 und WO 01/02017 A2 sind ebenso pegylierte Derivate des EPO bekannt, die neben der verlängerten Plasma-Halbwertzeit auch eine höhere klinische Wirksamkeit zeigen. Dazu werden beispielsweise durch gezielte Mutagenese 1 bis 6 zusätzliche Glykosylierungsstellen in die EPO-Aminosäuresequenz eingebracht. Auch dieses Derivat kann erfindungsgemäß eingesetzt werden.

Neben EPO und EPO-Derivaten mit entsprechender biologischer Funktion können auch andere hämatopoietische Wachstumsfaktoren wie beispielsweise TPO oder Teile davon erfindungsgemäß eingesetzt werden.

Bei dem TPO (bekannt auch als c-Mpl-Ligand, mpl-Ligand, Megapoietin oder Megakariozyten-Wachstums- und Entwicklungsfaktor) handelt es um einen hämatopoietischen Wachstumsfaktor mit einer komplexen biologischen Wirkung, der unter anderem die Entwicklung und Proliferation von Megakariozyten und Thrombozyten reguliert. Das reife TPO besteht aus einer Sequenz aus 332 Aminosäuren, wobei die N-terminale Region (RBD-Domäne) mit 154 Aminosäuren eine erhebliche Sequenz- und Strukturhomologie zu EPO aufweist (20 % Identität und weitere 25 % Ähnlichkeit). Insbesondere bildet das TPO zwei hoch konservierte Cysteinbrücken aus, die an homologen Positionen auch im EPO zu finden sind.

Es wurde in der Vergangenheit gezeigt, daß die N-terminale Region des TPO für die Bindung an den Cytokin-Rezeptor und die damit induzierte Signalkaskade über den JAK/STAT Weg verantwortlich ist (Geddis A.E., Linden H.M., Kaushansky K: Thrombopoietin: a pan-hematopoietic cytokine. Cytokine & Growth Factor Reviews 13 (2002) 61-73). Erfindungsgemäß kann daher auch nur ein Teil des TPO, insbesondere das N-terminale Fragment, verwendet werden.

Die Herstellung und Charakterisierung von TPO und dessen Varianten sind beispielsweise in EP 1 201 246, WO95/21919, WO95/21920 und WO95/26746 beschrieben. Diese werden zu Offenbarungszwecken vollumfänglich in Bezug genommen. Die Wirkung von TPO auf fötale Hepatozyten ist aus Untersuchungen von E. Schmelzer bekannt (E. Schmelzer: Optimierung der Kultur und Charakterisierung primärer embryonaler Hepatozyten der Ratte; Dissertation Göttingen 2002).

Als Varianten von TPO eignen sich beispielsweise die in der WO95/21919 beschriebenen TPO-Derivate oder die in WO95/21920 beschriebenen allelischen Varianten oder Spezieshomologe oder das in WO95/26746 und EP 1 201 246 beschriebene pegylierte TPO, ohne sie darauf zu beschränken. Als pegyliertes TPO wird im Sinne der vorliegenden Erfindung TPO-Derivate verstanden, die an ein organisches Polymer, wie z.B. Polyethylenglykol, Polypropylenglykol oder Polyoxyalkylen, gebunden sind. Als weitere Varianten von TPO werden auch Derivate von TPO verstanden, die eine Sequenzidentität von weniger als 100% besitzen und dennoch die Aktivität von TPO, wie vorzugsweise in EP 1 201 246 beschrieben, besitzen. Üblicherweise besitzen TPO-Derivate eine Sequenzidentität von mindestens 70%, vorzugsweise mindestens 75%, insbesondere mindestens 80% und vor allem mindestens 85% im Vergleich zu humanem TPO einschließlich derer Fragmente mit TPO-Aktivität. Eine besonders bevorzugte TPO-Aktivität im Sinne der vorliegenden Erfindung ist die Beschleunigung der Proliferation, Differenzierung und/oder Reifung von Megakaryozyten oder Megakaryozyten-Vorläufer in Plättchen-produzierende Formen dieser Zellen durch TPO oder dessen Varianten.

Erfindungsgemäß können EPO und TPO sowohl in ihrer humanen als auch in ihrer nicht-humanen Form eingesetzt werden. So zeigen beispielsweise humanes TPO mit Schweine- oder Maus-TPO eine Homologie von über 70%.

In einer alternativen Ausführungsform der Erfindung ist es möglich, dem Patienten nicht die hämatopoietischen Wachstumsfaktoren selber, sondern Faktoren zu verabreichen, die die Expression der Wachstumsfaktoren in den traumatisierten Geweben induzieren.

So ist beispielsweise von Naughton BA et al beschrieben (Age-related variations in hepatic regeneration and erythropoietin production in the rat, Am J Anat. 1977 Jul;149(3):431-8), dass Erythropoietin hauptsächlich in der Leber und Milz während neonataler Phasen gebildet wird und dass bei Nephrektromie das Phänomen der hepatischen EPO Bildung nach einer Hepatektomie wieder zunehmen kann. Diese Fähigkeit nimmt mit dem Alter ab. Später wurde dann erkannt (Hepatic regeneration and erythropoietin production in the rat.Naughton BA, Kaplan SM, Roy M, Burdowski AJ, Gordon AS, Piliero SJ. Science. 1977 Apr 15;196(4287):301-2. ), dass eine regenerierende Leber Erythropoietin als Antwort auf Hypoxie produziert. Es wurde gefunden, dass die EPO-Produktion abhängig ist vom Stadium der Leberregeneration, wobei die höchsten EPO Konzentrationen in der Phase des stärksten Leberwachstums gefunden wurden.

Dornfest et al. beschrieben 1981 (Recovery of an erythropoietin inducing factor from the regenerating rat liver. Dornfest BS, Naughton BA, Kolks GA, Liu P, Piliero SJ, Gordon AS. Ann Clin Lab Sci. 1981 Jan-Feb;11(1):37-46.), dass die Leber die Hauptquelle für die Bildung eines EPO-induzierenden Faktors ist. Bei Nephrektomie nahm die Bildung des Faktors in der Leber zu. Somit kann die erfindungsgemäße Wirkung des EPO auch mittelbar durch die Verabreichung eines Faktors erreicht werden, der die Expression des EPO induziert.

Ebenso kann die endogene EPO-Expression beispielsweise durch die Stimulierung der EPO Sekretion durch die mittel- oder langfristige Verabreichung des Wachstumshormons (Growth Hormone, GH) erreicht werden (Sohmiya, M., Y. Kato. Effect of long-term administration of recombinant human growth hormone (rhGH) on the plasma erythropoietin (EPO) and haemoglobin levels in anaemic patients with adult GH deficieny. Clinical Endocrinology (2001) 55, 749-754).

Die einzelnen Konzentrationen der Wachstumsfaktoren können in Lösung bei ca. 1 bis ca. 100 ng/ml, vorzugsweise bei ca. 10 bis ca. 50 ng/ml, insbesondere bei ca. 10 bis ca. 20 ng/ml liegen. Bei lokalen Beschichtungen (topischser Verabreichung) können die Konzentrationen der Wachstumsfaktoren jedoch auch ein Vielfaches davon betragen.

Die hämatopoietischen Wachstumsfaktoren können vorteilhafterweise auch für die Kultivierung von Gewebekulturen *in vitro* eingesetzt werden. Dazu werden die Zellen in für das Gewebe besonders geeigneten Vorrichtungen und Verfahren kultiviert, beispielsweise auf einem Gitter mit einer schneidenden Maschenstruktur von 500 m Größe, um immer wieder neue Tochteraggregate von Hepatozyten entstehen zu lassen. Insbesondere in vitro sind Kombinationen von EPO mit GH vorteilhaft.

Insbesondere können berührungslose, automatisch oder manuell gesteuerte Pumpsysteme eingesetzt werden, die z. B. aus Kolbenpumpen bestehen oder magnetisch bzw. durch Druckluftkompression von Schläuchen erzeugte, gerichtete Ströme erzeugen. In Anwesenheit von Endothelzellen kann es durch den Scherstress in einem perfundierten Bioreaktor zu einer spontanen Konfluenz der Endothelzellen auf den Oberflächen der Aggregate kommen, was für die weitere Verwendung vorteilhaft sein kann.

Für die Einkapselung eignen sich dem Fachmann bekannte, geeignete Materialien, in die beispielsweise strukturierte Formen oder Räume integriert werden, die eine *in situ* Wachstumsstruktur bzw. Vergrößerung ermöglichen. Alternativ kann auf die Kapsel verzichtet werden und beispielsweise in Gegenwart von Endothelzellen eine Endothelialisierung und somit eine optimale Hämokompatibilität erreicht werden.

Die erfindungsgemäße Gabe von EPO oder TPO führt entweder alleine oder bei grösseren strutkurellen Defekte auch in Verbindung mit scaffold Materalien zu einem biologischen Gewebeersatz. Diese Trägermateralien können in vitro bzw. extrakorporal vorbesiedelt sein, biologisch modellierbar (biodegradabel) sein und sind mikro- und makrostrukturell und biochemisch im Idealfall der zu ersetzenden Struktur möglichst ähnlich. Die biochemische Nähe bzw. Identität beinhaltet eine Rekontstruktion der in vivo Zusammensetzung durch Kollagene und Matrixproteine (Elastin, Fibronektin und alle Matrixkomponenten des Körpers in gewebespezifischer Prägung wie bekannt).
Stammzellen können aus den verschiedenen im Körper des Patienten vorhandenen Quelle erhalten werden: Knochenmark, peripheres Blut, Fettgewebe, dem Gewebe selbst, Nabelschnurblut- bzw. Gewebe. Allogene Stammzellen können entsprechend gewonnen werden, sind aber mit immunologischen Nachteilen behaftet. Embryonale Zellen können verwendet werden, sind aber mit den entsprechenden Nachteilen behaftet.

In einer besonders bevorzugten Ausführungsform kann der strukturierte Wachstumsprozess der kultivierten Zellen durch eine mit den erfindungsgemäßen Wachstumsfaktoren beschichteten Matrix induziert werden. Dazu kann die Matrix mit einem oder mehreren der genannten Wachstumsfaktoren sequenziell behandelt werden.

Sofern es sich um eine biologische Matrix handelt, ist es üblicherweise ein Implantat (Stent, Patch oder Katheter), ein Transplantat (z. B. Hauttransplantat), ein Trägermaterial zum Wachstum von Zellen, z.B. ein sogenanntes Slowrelease Material (z.B. ein Hydrogel auf der Basis von Fibrin und/oder Polymere, wie z. B. Polylaktid oder Polyhydroxyalkanoat, und/oder Alginate), ein Knochenersatzmaterial (z.B. Tricalciumphosphat), ein allogenes, autologes oder xenogenes azellularisiertes oder nicht-azellularisiertes Gewebe (z.B. Herzklappe, Venenklappe, Arterienklappe, Haut, Gefäß, Aorta, Sehne, Comea, Knorpel, Knochen, Trachea, Nerv, Miniskus, Diskus intervertebralis, Ureteren, Urethra oder Blase (siehe z. B. EP 0 989 867 oder EP 1 172 120)), oder eine Matrix (z.B. eine Laminin-, Collagen IV- und/oder Matrigel-Matrix) oder vorzugsweise ein Feeder-Layer, wie z.B. Collagen I-, 3T3- und/oder MRC-5-Feeder Layer, oder ein Collagenfließ.

Die biologische Matrix wird vorteilhafterweise mit gewebespezifischen Zellen, Vorläuferzellen, Knochenmarkszellen, peripheres Blut, Fettgewebe und/oder Fasergewebe, z.B. mit adulten Vorläuferzellen aus dem Knochenmark, vorbesiedelt. Die Vorbesiedelung führt dazu, dass der Regenerationsprozess bereits *in vitro* einsetzt damit nach der Implantation der Matrix in den Körper die Regenerationszeit *in vivo* verkürzt ist.

Die verwendeten Matrices können noch zuvor aktiviert werden. Die Aktivierung der biologischen Matrix oder Trägerstruktur kann beispielsweise mittels Plasmaionisation, z.B. unter Verwendung von Wasserstoffperoxid, oder mittels Laseraktivierung erfolgen.

Alternativ kann eine Beschichtung mit einer biodegradablen (Bio)polymerschicht, die den oder die genannten Wachstumsfaktor(en) enthält, vorgenommen werden. Hierzu eignen sich beispielsweise Fibrin, Plasma, Blut, Collagen und/oder Polylaktide.

Das erfindungsgemäße Verfahren eignet sich insbesondere für adulte Zellen, d. h. primär differenzierte Zellen, die keinen embryonalen oder fötalen Phänotyp mehr besitzen. Es eignet sich insbesondere für humane adulte Zellen, beispielsweise für adulte Progenitorzellen, gewebespezifische Zellen, vorzugsweise Osteoblasten, Fibroblasten, Hepatozyten und/oder glatte Muskelzellen.

Neben - einer Beendigung bzw. Reduzierung der Zufuhr der beschriebenen Wachstumsfaktoren zu der Kultur eignen sich zur Terminierung des erfindungsgemäßen Wachstumsprozesses auch Somatostatin und/oder TGF beta und/oder Prostaglandine.

Es ist besonders vorteilhaft, dass erfindungsgemäße Verfahren lokal *in vivo* einzusetzen. Dazu können die Wachstumsfaktoren z.B. auf die Resektionsfläche eines Organs (z. B. einer Leber) aufgebracht werden. Sie können topisch oder aber über mit Hilfe eines Katheters lokal oder systemisch appliziert werden. Im Falle einer Leberresektion können sie alternativ oder ergänzend vor, während oder nach dem Eingriff appliziert werden. Ebenso ist es möglich, die Wachstumsfaktoren (z.B. zur Förderung der Knorpelregeneration) unmittelbar in das betroffene Gewebe oder Gelenk zu injizieren. Damit können die Wachstumsfaktoren über die Synovialflüssigkeit direkt auf die Bildung einer neuen Knorpelstruktur wirken.

In einer besonderen Ausführungsform können zusätzlich zu den hämatopoietischen Wachstumsfaktoren einer oder mehrerer der folgenden Wachstumsfaktoren verabreicht werden: "Transforming Growth Factor beta" (TGF beta), Prostaglandine, Granulozyten (-Makrophagen)-stimulierender Faktor (G(M)-CSF), "Growth Hormone Releasing Hormone" (GHRH), "Thyrotropin-releasing Homone" (TRH), "Gonadotropin-releasing Hormone" (GnRH), "Corticotropin-releasing Hormone" (CRH), Dopamin, "Antidiuretic Hormon" (ADH), Oxytocin, Prolactin, Adrenocorticotropin, beta-Celltropin, Lutrotropin und/oder Vasopressin verwendet bzw. zusätzlich ein oder mehrere Nervenregenerationsfaktoren, vorzugsweise "nerve growth factor" (NGF) und/oder ein oder mehrere Gefäßregenerationsfaktoren, vorzugsweise "Vascular Endothelial Growth Factor" (VEGF) und/oder "Plateled Derived Growth Factor" (PDGF).

Die hämatopoietischen Wachstumsfaktoren parenteral als injezierbare Mikrosphären, erodierbare Partikel, Polymerverbindungen (mit Polypeptid, Polyglycolsäure), Liposomen und kleine Partikel appliziert werden. Injezierbare oder implantierbare Drug Delivery ―Geräte sind ebenso möglich. Die Stoffe können auch inhaliert werden, subkutan, intramuskulär gespritzt werden oder ― für die topische Applikation - als kutanes Pflaster gegeben werden.

Begleitend können Substanzen mit den Pflastern vermischt werden, die eine lokale Hyperämie erreichen und dadurch die Hautpermeabilität erhöhen (z.B. Bienengift). Hyperionische Strukturen mit und ohne direkte EPO Kopplung bzw. Beschichtung der Salze können durch die Hautbarrieren besser transportiert werden und können mit bevorzugt positiv ionisierenden Strukturen verbunden werden. Auch negative Ionisation ist möglich. Die Faktoren können mit Mandelöl bzw. Jojobaöl zur Transportverbesserung durch Schleimhäute im Darmbereich bzw. der Haut verwendet werden.

Eine Verbindung mit Polyethylenglykol (PEG) ist möglich, um Transportbarrieren (Mucosa im Mund, Magen, Darm; Schleimhäute, Hornhaut) zu überwinden.

Es ist bekannt, dass aus Proteinen Mischpräparationen vorbereitet werden können. Suspensionen, Gelimulsionen, Feststoffverbindungen, dehydrierte oder lyophilisierte Puder sind möglich. Die Wachstumsfaktoren können auf Partikeln absorbiert werden oder enkapsuliert werden.

Es kann besonders vorteilhaft sein, Stammzellen (Progenitoren im eigentlichen Sinne) gemeinsam mit EPO / TPO zur Gewebregeneration einzusetzen, um damit das Recruitment der Gewebezellen aus den 4 Grundgewebearten für den Regenerationsprozess deutlich zu beschleunigen. EPO / TPO sowie die anderen genannten Faktoren können gemischt mit Stammzellen und z.B. Fibrinkleber als Trägermatrix appliziert werden. Bei Bedarf kann die Trägermatrix weggelassen werden bzw. durch eine stärker vorstrukturierte und geformte Trägermatrix ersetzt werden. Die Faktoren können auch ohne jegliche biologische Trägermatrix z.B nur in wässrigrer Suspension systemisch oder topisch verabreicht werden.

Die erfindungsgemäße Gabe von EPO verbessert diese Geweberegeneration durch gewebespezifische Prägung der Stammzellen und Differenzierung im Anschluss an eine Vermehrung und Integration und koordiniert das Wachstum in Bezug auf die Grundgewebearten.

### I. Anwendungsbereiche

### 1. Plattenepithelien

Erfindungsgemäß wird EPO eingesetzt, um die Ausbildung von Basalmembranen (extrazelluläre Stützschicht unter Epithel) zu fördern. EPO unterstützt die Ausbildung des Grenzbereiches der Papillen.

EPO wird erfindungsgemäß eingesetzt, um die Ausbildung von Drüsenepithel zu unterstützen. EPO führt zur Regeneration plattenförmiger isoprismatischer und prismatischer Epithelien (Plattenepithelium Ösophagus, hochprismatisches Epithel im Samenleiter, Übergangsepithel der Harnblase).

Durch EPO kommt es im strukturellen Wachstumsprozess zur Wiederausbildung der Desmosomen, d. h. der Verbindungsstücke zwischen den Zellen und deren Anheftungsstrukuren. Es kommt auch zur Ausbildung von sogenannten Mikrovilli auf den Epithelien, zum Beispiel des Dünndarms (Nebenhoden, Luftröhre).

EPO hat einen direkten Effekt auf die Regeneration des einschichtigen Plattenepithels, der Endothelauskleidung von Herz-, Blut- und Lymphgefäßen und des hochprismatischen Epithels (Magen, Dünn- und Dickdarm, Gallenblase, Eileiter, Uterus). EPO wird erfindungsgemäß eingesetzt, um das zweireihige Epithel (Speicheldrüsen der Mundhöhlen, Tränennasengang, Nebenhodengang, Samenleiter) auszubilden. EPO wird erfindungsgemäß auch für das hochprismatische Epithel (Nasenhöhle, Epipharinx, Kehlkopf, Luftröhrenbronchialbaum, Harnröhre, Ohrtrompete), weiter zur Ausbildung von unverhorntem und verhorntem Plattenepithel eingesetzt. Der strukturfördernde Aspekt auf die Parenchymregeneration des EPO unterstützt die Ausbildung exokriner Drüsen.

Hierzu zählen unter anderem das Pankreas, Drüsen im Dünndarmepithel (Becherzellen, Unterkieferspeicheldrüse, Inselzellen zur Insulinproduktion). EPO unterstützt die Regeneration der endokrinen Drüsen.

### 2. Bindegewebe

EPO kann die Grundbestandteile des Bindegewebes in Koordination mit den umgebenden Parenchymdrüsenstrukturen wiederherstellen. Die Grundbestandteile, die hiervon beeinflusst werden, sind die Grundsubstanz (Glucosaminoglycane), die Koordination mit sogenannten Plasmazellen, Fettzellen, Blutgefäßen und darum liegenden glatten Muskelzellen, daneben die Bindegewebszellen, Fibroblasten, Mastzellen und Kollagenfasern sowie die Elastinfasern und dazwischen eingefügte Makrophagen- (ebenso Kupffer, Pit, Itozellen bei der Leber) und Kapillarenendothelzellen. Kapillaren haben, nur eine Endothelzellenauskleidung.

Hierbei hat EPO jedoch einen breiteren koordinativen Aspekt auf das geschilderte Mikromilieu. Dieses wird durch traumatische Verletzungen in einen Initiatorzustand versetzt. Therapeutisch kann EPO erfindungsgemäß gegeben werden, wenn nach traumatischen Verletzungen diese Initiationskaskaden beschleunigt werden sollen. EPO unterstützt hierbei erfindungsgemäß als Katalysator und systemischer und topischer Vermittler der Geweberegeneration den durch Makrophagen regional ausgelösten immunmodulatorischen Effekt, der in Makrophagen in Verbindung mit Plasmazellen die immunologische Modulation und Freisetzung von Interleukinen und Zytokinen beinhaltet. Durch diese erfindungsgemäße Interaktionskompetenz wird die hohe Regioselektivität der therapeutischen Gabe von EPO / TPO am Bedarfsort (Trauma, Entzündung, Automimmunerkrankung) erreicht.

EPO koordiniert in der therapeutischen Anwendung erfindungsgemäß diese regionale Immunstimulation mit der systemischen endogenen Antwort. EPO fördert dadurch das Anlaufen der Entzündungsreaktion im Sinne einer Kaskadenbeschleunigung als therapeutischen Effekt. Es erhöht die Freisetzung von endogenen Wachstumshormonen und kontrolliert damit auch dessen Nebenwirkungspotential. Eine systemische Gabe von Wachstumshormonen wird dadurch vermeidbar und dessen unkontrollierter Effekt bezüglich einer potentielle Tumorogenizität im Rahmen der physiologischen Wirkungssituation vermieden.

Bindegewebsstrukturen können erfindungsgemäß wiederhergestellt werden. Hierzu zählen kollagene Fasern, retikuläre Fasern, zum Beispiel Lymphknoten, elastische Fasern und die Ausbildung der Grundsubstanz (Proteoglycane, Glycosaminoglycane, Hyaluronsäure, Chondroitinsulfate, Dermatansulfat, Keratansulfat, Heparansulfat, Heparin). Die modulierten Bindegewebszellen sind die Fibroblasten, Retikulumzellen, insbesondere in lymphatischen Geweben und Organen, Mesenchymzellen, Fettzellen, Monozyten und Makrophagen. Es ist bekannt, dass Monozyten sich zu Makrophagen differenzieren können. EPO kann die Makrophagenabwehr in der Entzündung und bei Infektionsreaktion hinsichtlich der nachfolgenden und notwendigen Gewerbereparatur und 3-D-Regeneration beschleunigen.

Die chemotaktischen Funktionen der Makrophagen werden durch EPO mitkoordiniert. Die ortsständigen, im Bindegewebe befindlichen Monozyten können in das Geweberecruitment durch EPO hineingezogen werden, wodurch diese im Sinne von systemisch und lokal verfügbaren Progenitoren und für eine ortsständige Reparatur direkt beitragen können.

EPO koordiniert die Funktion des retikoenterialen Systems (RES). Dieses wird in einem Netzwerk retikulärer Fasern zusammengeschlossen und bezieht die Endothelzellen von Leber, Milz und Knochenmark mit ein. Daneben werden durch EPO die Lymphozytenaktivität, die der Plasmazellen, Makrophagen und - immunmodulatorisch - die der Mastzellen koordiniert.

Im Anschluss an traumatische Vorgänge koppelt EPO die an sich bekannten Einzelfunktionen dieser Zellen mit dem Strukturaufbau und der regioselektiven Geweberegeneration. Danach kommt es über Rückopplungseffekte zum Abklingen der Entzündungsreaktionen und damit auch zu einem Abklingen von Autoimmunphänomenen, die auch über Antikörperbildung vermittelt werden können. Hierbei wird EPO erfindungsgemäß therapeutisch als Medikament genutzt.

Bei der Geweberegeneration kommt es zur Ausbildung von Bindegewebsstrukturen und dem Aufbau lockerer Bindegewebsstrukturen (Subkutis der Haut, bei Hohlorganen, wenig spezialisiertes Bindegewebe). EPO führt zur Ausbildung des Bindegewebes (ungeordnetes und geordnetes Bindegewebe, der Ausbildung in Bändern, Ligamenten, Faszien, Sehnen und der Koordination mit elastischen Strukturen und deren dreidimensionaler Vernetzung, die gewebespezifisch vermischt oder auch gallertartig ausgeprägt wird).

EPO unterstützt den Aufbau der Retikulumzellen, die insbesondere das retikuläre Bindegewebe wiederherstellen. EPO unterstützt den Aufbau des Fettgewebes und der darin eingeordneten Progenitoren durch Recruitment und Einsatz der Propagationsfunktionen des EPO.

Durch die erfindungsgemäße Wirkung kommt es zur Verbesserung der Stützfunktionen und der Kraftübertragung dieser Bindegewebskomponenten. Es kommt zur Verbesserung des Stoffaustausches, da das Bindegewebe Körpergefäße führt. Es kommt zur Verbesserung der Speicherfunktionen der Bindegewebsstrukturen mit einer Einlagerung von Lipiden, Glycosaminoglycanen. EPO führt therapeutisch zur Verbesserung der Wasser- und Elektrolytfunktion.

Es kommt durch EPO zur Verbesserung der Schutzfunktion, da es zum Wiederaufbau mechanischer Barrieren gegenüber pathogenen Krankheitserregern kommt. Es kommt zu reparativen Prozessen im Anschluss an Entzündungen, da die Gewebsstrukturen wiederhergestellt werden. EPO unterstützt erfindungsgemäß die Parenchym- und Strukturregeneration.

Dies ist im Unterschied zu sehen mit der Ausbildung von Granulationsgeweben (wie diese für eine sogenannte Wundheilung charakterisierend sind), die im Sinne einer Wundheilung zu Ersatzgeweben und damit lediglich zu Ersatzfunktionen führen. Hierunter versteht man die Ausbildung von Narbengeweben und lockeren fasrigen Geweben, die eine Defektdeckung erreichen können, die jedoch mit hohen funktionellen Verlusten verbunden sind. Erfindungsgemäß besitzt EPO hier einen strukturbildenden Prozess im Sinne der Wiederherstellung des ehemaligen Gewebes. In besonders grossen Defekten können Kombinationen mit Platzhaltermaterialien jedoch den strukturbildenden Prozess unterstützen und fördern (morphologisch und funktionell). Dies hat grosse Bedeutung für das Tissue Engineering, da jegliche zur Integration verwendeten scaffold Materialien (z.B. Trägermaterialien für Gefäße, Herzklappen, Transplantate) mit EPO kombiniert werden können. Permanente Implantate (nicht biodegradable Materalien) können zur besseren Gewebeintegration beschichtet werden (z.B. metallische und keramische Prothesen).

### 3. Blutgefäß- und Lymphkreislaufsystem

EPO führt zu einer Wiederausbildung des Blutgefäßsystems und auch der großen Blutgefäße - nicht nur der Kapillarstrukturen, wie bisher bekannt. Zu den Strukturen der großen Blutgefäße gehören auch die Venen und Arterien (grosser und kleiner Körperkreislauf).

Weiterhin ermöglicht EPO Regenerationsprozesse innerhalb von Gewebestrukturen, die auch das Lymphkreislaufsystem betreffen, und der Lymphgefäße selbst. Die Wiederherstellung durch EPO-Integration betrifft nicht nur die Tunica Intima, das Endothel, sondern vor allem auch die Tunica Media, die aus Bindegewebe und Muskelzellen besteht. Weiterhin die Tunica Externa, die eine längslaufende Bindegewebslage bildet. Wichtig bei der Ausbildung der Gefäße sind die verschiedenen Typen der Arterien. So gibt es Arterien vom elastischen muskulären Bautyp und unterschiedliche Bindegewebsstruktur, die durch regenerative Prozesse mitinduziert werden, nämlich die Membrana Elastica Interna und die Membrana Elastica Externa, sowie die Ausbildung von vasovasalen Gefäßen. Hierbei werden Fettzellen in die Wandschichten integriert. Bei den Arterien vom elastischen Bautyp ermöglicht EPO die Regeneration der Membrana Elastica Interna neben der Tunica Externa mit den Vasovasolen. Bei den Arterien vom muskulären Bautyp, Querschnitt 0,1 bis 7mm, ermöglicht EPO eine kräftige Ausbildung der glatten Muskulatur in der Media. Weiterhin ermöglicht EPO die Ausbildung der subendothelialen Bindegewebslage. Es kommt zur Wiederherstellung typischer Aufbauformen, wie der Tunica Intima, wie bisher bekannt als Endothelzell-Komponente, aber auch vor allem der Membrana Elastica Intema, den elastischen Phasenstrukturen, Muskelzellen, Tunica Media, Membrana Elastica und Tunica Extema. Auch räumlich geometrische Anordnungen wie die Ausbildung muskulärer Venolen im Bereich begleitender Venolen in einer spezifischen geometrischen Anordnung der Nachbarschaft werden durch die therapeutische EPO Gabe ermöglicht.

Es handelt sich nicht um 2-dimensionale Verbindungsprozesse mit einem Konglomeratbildungs-Schub, sondern um spezifische 3-dimensionale Strukturbildungsprozesse. EPO und TPO sind erfindungsgemäß somit nicht mit den bekannten Proliferationsfaktoren im Sinne klassicher Wachstumsfaktoren gleichzustellen, sondern wirken eher im Sinne "3-D Regenerationsfaktoren".

Ähnliche Ergebnisse finden sich im Bereich der EPO-Stimulation bei Venen mit der Ausbildung der Tunica Intima, Tunica Media und Tunica Adventitia. Weiterhin können die Venenklappenstrukturen insbesondere in Verbindung mit scaffold Materialien in geometrische Formen regeneriert werden, oder auch scaffolds soweit dies in größeren Gefäßbereichen nach traumatischen Verletzungen und degenerativen Prozessen möglich ist.

Bei größeren Volumendefekten können unter Umständen Trägermaterialien mit implantiert werden, so dass die EPO-Wirkung auf den Remodelling-Prozess und die Integration in den Gesamtorganismus fokussiert wird. Bei kompletten regenerativen Prozessen vor allem bei parenchymatösen Strukturen werden diese Gefäße jedoch mit gebildet.

Es gibt keine Beschränkung auf verschiedene Gefäßstrukturen, z.B. arterivenöse Anastomosen. In Verbindung mit einer therapeutisch gegebenen EPO-Gabe können insbesondere auch altersbedingte Veränderungen der Blutgefäße und degenerative Strukturen revidiert werden. Im Alterungsprozess kommt es zur Ausbildung glatter Längsmuskulaturstrukturen. Degenerative Prozesse bedeuten nach dem Bauprinzip der Gewebsstrukturen angelegte Neostrukturbildungsaktivitäten. Diese können als de novo hergestellte Gefäße in Gewebsstrukturen aufgebaut werden und sind nicht primär pathologisch verändert.

Die Entzündungsreaktionen können mittels EPO-Gabe erfindungsgemäß modelliert werden. Mittels EPO-Gabe gibt es Regenerationsprozesse im Bereich des Endokards wie auch des Myokards. Das Epikard ist ein Blatt des Herzbeutels, das durch EPO-Gabe in eine Regeneration geführt wird. Das parietale Blatt Perikard stellt eine seriöse Membran dar, die aus einer Mesothelzellschicht, die auf einer Bindegewebslage ruht, besteht. Im Bereich des Herzens kommt es zu einer Regeneration der Herzventile und auch der Chordae tendinäe insbesondere dann, wenn Strukturhilfen (scaffolds) in Verbindung mit Stammzellbesiedelung und EPO-Gabe herbeigeführt werden. Die Wiederausbildung des Reizleitungssystems kann nach regionaler Zerstörung erfolgen. Hierzu zählen auch Purkinjefasern, sowie der Atrioventikulär-Knoten, der auch repariert werden kann. Zusätzlich werden Lymphgefäße, Nerven, Herzgeflechte, Koronaarterien-Strukturen miteinander kombiniert.

Der Aufbau der Lymphgefäße und Lymphkapillaren erfolgt durch EPO-Gabe entsprechend der normalen Form- und Sammelgefäße (Tunica Intima, Tunica Medica und Tunica Adventitia). Regenerative Prozesse können auch den Bereich des Ductus thoraticus betreffen.

Klappen- und Sammelgefäße stellen Falten dar, die ebenso bei strukturellen Aufbauprozessen mitintegriert werden. Beim Blutgefäßsystem kommt es zu einer Aktivierung von Angioplasten, die zum Teil aus peripheren, aber auch regionalen Progenitoren rekrutiert werden. Zunächst kommt es zur Anlage von Kapillaren, die aus Endothel-Zellstrukturen bestehen. Wichtig im Anwendungsbereich von EPO ist jedoch, dass der Strukturaufbau initiiert werden kann, indem die nachgeschalteten muskulären Strukturen und Bindegewebsstrukturen aufgebaut werden.

Das Reizleitungssystem spielt eine große Rolle für die Koordination der EPO Wirkung, da es Interaktionen zu den modulierenden Zellen und Knoten gibt.

Zu den Zellen des Immunsystems gehören Lymphozyten, Makrophagen, Plasmazellen, Stammzellen. Das Maschenwerk retikulären Bindegewebes verbindet die Zellen des Immunapparates im Immunsystem. Durch EPO-Gabe kommt es zu einer beschleunigten Reparatur des retikulären Bindegewebes, der Retikulumzellen und der Retikulumfasern. Ebenso können Thymusstrukturen wieder repariert werden. Histologisch werden dort Rinden- und Markstrukturen wiederhergestellt. Histologisch kommt es bei der EPO-Gabe zu einer Regeneration auch von Lymphknoten, die wiederum Intermediärsinus, Marksinus-, Randsinus-, Kapsel- und Bindegewebssepten, aber wie auch abführende Lymphgefäße, Lymphknötchen oder Marksträngen ausbilden können.

Die histologische Organisation entspricht nach erfolgtem Reparaturprozeß und der koordinierten Zusammenfassung einer üblichen Struktur mit Kapsel- und Bindegewebssepten, Hilusbindegewebe und zuführenden/abführenden Lymphgefäßen, Lymphknötchen, Marksträngen und abführenden Lymphgefäßen.

Regenerative Prinzipien können auch im Bereich der Milz zu einer Wiederherstellung der normalen Kapsel, der Partikelstrukturen und des Hilus führen. Weiterhin finden wir für die Wiederherstellung der weißen Pulpa Malpighikörperchen der Milzsinusplatten in den Milchsträngen. Der Blutkreislauf der Milz wird wiederhergestellt, wobei Arterien der Milz, Milzsinusvenen und Pulpavenen zusammen geführt werden. Die weiße Pulpa und die Verbindung der Lymphscheiden wird wiederhergestellt.

Durch EPO Gabe nach Verletzungen kommt es zu einer dreidimensionalen Interaktion der Basalmembranen, der Retikulumzellen, der Retikulumfasern und der Erythrozyten. Es kommt zur Ausbildung offener und geschlossener Kreislaufsysteme. Hierbei ist die Interaktion mit Makrophagen, die aus Knochenmarkszellen entstehen können, von Bedeutung, um endogene Kaskaden innerhalb des Körpers und der Vermittlung von Wachstumsmediatoren (Wachstumshormon) initiieren zu können.

### 4. Haut/Cutis

Reparative Prozesse nach Verbrennungen, Trauma, Verbrühungen, mechanischen Verletzungen oder Entzündungen im Bereich der Haut sind sehr vielfältig. Durch Gabe von EPO oder TPO kann mit und ohne strukturelle Ersatzstrukturen eine Regeneration erreicht werden.

Insbesondere bei chronischen Erkrankungen, Durchblutungsstörungen, diabetischen Ulzera, Phänomenen auch immunologischer Natur kann EPO hier modellierend eingreifen. Histologisch gesehen kommt es zu einer Wiederausbildung einer normalen Epidermis, Dermis und einer Subkutis, die eine entsprechende Vaskularisation mit Makrostrukturen erreicht. An den Handflächen und Fußsohlen kommt es zur Ausbildung des stratum basale, stratum granulosum, stratum lucidum, stratum coneum. An der Dermis und der Außenseite kommt es zur Ausbildung von Dermispapillen. An der Epidermis (innere Oberfläche) werden die entsprechenden Epidermisvertiefungen wieder ausgebildet. Zusätzlich werden Anhangsgebilde und deren Ausbildungen aus Progenitoren mitunterstützt. Ein Problem beim bisherigen Tissue Engineering war die Verbindung struktureller Reparatur mit der Ausbildung von Hautanhangsgebilden im Bereich der Haut.

Durch Gabe von EPO können Interaktionsprozesse im Bereich des Melanozytensystems des Menschen erreicht werden. Physiologische Rückkopplungsprozesse zwischen Augen, Hirnhaut und der Ausbildung der Pigmentierung werden berücksichtigt, da es sich hier nicht um isolierte Zellverbindungen, sondern organtypische Regenerationsprodukte handelt.

Reparative Prozesse des dermalen Anteiles beinhalten muskuläre Strukturen (musculus arector pili), taktile Strukturen, äußere Wurzelscheide der Haare und innere Wurzelscheide und auch die Haarzwiebelstruktur. Diese ragt unter Umständen in das Fettgewebe hinein. Bei den Haaren und Haarfollikeln kommt es zur Ausbildung von bindegewebigen Wurzelscheiden, die eine wichtige Funktionsstruktur in der Regeneration beinhaltet. Daraus bilden sich Glashaut, äußere Wurzelscheide, innere Wurzelscheide und das letztendliche Haar. Ähnliche Prozesse können im Bereich der Nagelregeneration unterstützt und herbeigeführt werden. Innerhalb der Gewebestrukturen werden Schweißdrüsen mit ausgebildet. In den Hautstrukturen werden perifollikuläre Netzstrukturen, Netze, subkapilläre Arteriennetze, termale Venennetze wieder ausgebildet.

### 5. Verdauungssystem

Durch die EPO-Gabe kommt zur Wiederherstellung der innersten Schicht der Schleimhaut, Tunica mukosa und der Bindegewebsschicht der sogenannten Lamina propria. Die tiefste Schicht bildet eine Lage glatter Muskulatur die Lamina muskularis mukosae. Der Schichtaufbau wird wie folgt: Serosa-Zellen dann Muskularis, Submukosa und Mukosa. Diese Strukturen werden durch Verdauungsdrüsen durchzogen und Schleimhautdrüsen befinden sich in der Mukosa. Zusätzlich regenerieren Submukosadrüsen und der Plexus myintericus (Auerbach) und der Plexus submukosa (Meißner). Im Bereich der Zunge regenerieren die blattförmigen Papillen. Im Bereich der Mundes werden im Bereich der Gewebsregeneration auch Geschmacksknospen mit regeneriert. Diese sind aufgebaut aus Basalzelle Typ 4, Stützzelle Typ 1, Rezeptorzelle Typ 3, Glykoproteine und Geschmacksporen. Die seromuköse Speicheldrüse besteht aus Streifenstücken, Schaltstücken, mukösen Endstücken, Basalmembranen, myoepitelialen Korbzellen und serösen Endstücken. Wichtige reparative Prozesse können bei Paradontose auch im Bereich des Zahnbereichs erfolgen. Zum Beispiel betrifft das die Strukturen des Periodontiums in Verbindung mit dem Alveolarknochen und dem Wurzelkanal mit Apex. Die reparativen Prozesse können durch Hinzuführung von stammzellbesiedelten Calziumphosphatstrukturen oder anderen mineralischen Ersatzmaterialien, die mit EPO beschichtet werden, erreicht werden. Die von EPO unterstützte Ausbildungen eines Regenerationsprozesses erfolgt im Bereich von Zysten im Bereich der Kieferhöhlen, Unterkieferstrukturen, nach Wurzelentfernung und Neureparatur dieser Strukturen vor Ort *in vivo*.

Die entsprechenden Materialien und Odontoblasten können *in vitro* ebenso durch EPO- Gabe vorbereitet werden.

Osteochondrale Strukturen wie zum Beispiel der osteochondrale Zylinder können in vitro vorbereitet und vivo unter EPO-Gabe wachstumsregenerationsfördemd implantiert werden. Die Ausbildung der Wurzelkanalstrukturen mit Neoinnervation wird über EPO beschichtete Leitstrukturen, die hineingegeben werden, gefördert. Hier helfen Moleküle der extrazellulären Matrix, die in Beschichtungsprozessen auch als Peptidstrukturen herbeigeführt werden können. Die Schmelzorganstrukturen zusammen mit Pulpaodontoplasten, Zwischenzonenschmelz-Pulpa können im Wachstumsprozess nach Verletzungen und traumatischen Prozessen unterstützt werden. Die reparativen und regenerativen Prozesse fokussieren sich auf den Bereich des umgebenden Bindegewebes und die Integration der Zahnstruktur oder auch eines Implantates im Bereich des Kiefers. Das Pulpa-Bindegewebe und die Ausbildung mit Blutgefäßen, die den Apex des Wurzelkanals ein- und ausdämmen, spielt hier eine große Rolle für die Innervation. Das Peridontium (Wurzelhaut) wird so regeneriert. Die Sharpeyfasern durchziehen die Wurzelhaut und sind im Zement bzw. Alveolarknochen verankert.

EPO führt zur regenerativen Prozessen in diesem Bereich nach Entzündungen und traumatischen Reaktionen. Im Bereich des Zahnfleisches kommt es durch EPO-Gabe bei Entzündungsphänomen bei Paradontose zur Verbesserung des reparativen Prozesses.

### Schlund:

Regenerative Prozesse können auch durch EPO-Gabe auf den Pharynxbereich in der Wiederherstellung des plasmatischen Mehrreihenepithels ausgedehnt werden. Auch hier können Tunica muscularis und Tunica adventitia koordiniert wiederhergestellt werden. In den Lymphozytenstrukturen innerhalb der Gaumenmandel werden Kryptastrukturen und Kapselstrukturen wiederhergestellt. Regenerative Prozesse können auch entzündete Strukturen, wie bei Tonsillitis regioselektiv in der Regeneration fördern.

### Rumpf/Darm:

Im Darmbereich gibt es hochregenerative Epithelanteilen, die sich innerhalb von 24 bis 48 Stunden erneuern können. Bei Entzündungsreaktionen kommt es zu einer Störung dieser reparativen Tätigkeit, so dass unterstützende Effekte durch EPO/TPO hier eine Kompensationsreaktion des Körpers ermöglichen können. Hierdurch kommt es zur Wiederausbildung von Epithelstrukturen wie Lamina propria, Tunica mucosa; Lamina muscularis mucosae , Tunica submuscosa, Ringmuskulatur, Tunica muscolaris und Längsmuskulatur und Tunica adventitia, die als reparative Prozesse den gesamten Darmwandbereich durchziehen würden, so dass eine selektive, oberflächliche auch intramurale und tiefmurale Regeneration ermöglicht wird. Diese Prinzipien können über den gesamten Bereich des Magen-Darm-Traktes beginnend von Schlund, Mund bis in die Speiseröhre und in den Enddarm fortgeführt werden. Im Ösophagus können die Lamina muscularis mucosae, Lamina propria und das mehrschichtige Plattenepithel regeneriert werden. Seröse Strukturen werden integriert und durch EPO Gabe repariert.

Der Wiederaufbau der Magenwand insbesondere der Bereich der Areae gastrici mit Magengrübchen, Magendrüsen, Lamina muscularis mucosae, an den Außenseiten Mesothel, dann subseröses Bindegewebe, Tunica muscolaris, Tunica submucosa und Tunica mucosae ist möglich. In den Strukturen werden Belegzellen, Magendrüsen, Hauptzellen, Lamina propria, muscularis mucosae und Tunica mucosae regeneriert, ebenso das Oberflächenepithel. Regenerative Prozesse auch im Bereich der Zellen des Corpusfundus-Drüsenbereiches beinhalten die Wiederausbildung von Hauptzellen. Die Verbindung zu Belegzellen ist gegeben. Die durch EPO erfindungsgemäß erreichte Stimulation beinhaltet auch die Regeneration von Zellen die vasoaktive Substanzen wie Polypeptide freisetzen und die Bildung der enterochromaffinen Zellen (ECN), die Serotonin freisetzen. Magendrüsen können regeneriert werden. Es ist bekannt, dass sich das Oberflächenepithel des Magens innerhalb von drei Tagen erneuern kann. Bei starken traumatischen, entzündlichen Verletzungen, Regenerationsstörungen hilft die Gabe von EPO diese Regenerationsfähigkeit wiederherzustellen. Im Bereich des Dünndarms kommt es zur Wiederausbildung der Lamina propria, der Lamina muscolaris mucosa, der Solitärfollikel, der Tunica submucosa, der Ringmuskulatur, der Längsmuskulatur, der Tunica serosa. In den Strukturen werden die Darmzotten wieder ausgebildet und ebenso das Solitärfollikel und die Kerkringschen-Falten. Ebenso werden die Krypten wiederhergestellt. Die Krypten enthalten sogenannte Panethzellen. Diese können Sekretgenerat im Drüsengrund ausbilden. Peyerscheplaques befindet sich in der Lamina propria des Duodenums. Die Endstücke der Brunnerschen Drüsen zeigen nach EPO Regeneration charakteristische muköse Zellen, ein helles Zytoplasma und an der Zellbasis liegende flache Zellkeme. Die Dickdarmregeneration bei EPO Gabe spielt insbesondere nach operativen Entfernungen eine wichtige Rolle.

### 6. Skelett

EPO führt zur Regeneration des Skelettgewebes mit der Unterteilung in straffes collagenes Bindegewebe, Knorpelgewebe und Knochengewebe. Bei den Chondrozyten fördert EPO die Ausbildung der interzellulären Substanzen und führt zu funktionellen Verbesserungen, der Chondrozytenstrukturen. EPO unterstützt die Ausbildung der Wachstumszonen und die Ausbildung des Perikondiums (umgebende Bindegewebsstruktur, Ausnahme Gelenk) nach traumatischen Effekten. Bei Erwachsenen können sich Knorpelzellen bekannterweise nicht mehr vermehren, da die Knorpelbildungsaktivität des Perikondiums auf die Zeit des Körperwachstums vor dem Erwachsenenalter beschränkt ist. Durch Gabe von EPO können diese Regenerationsprozesse therapeutisch beeinflusst und die ansonsten auftretende Bildung von Bindegewebszellen als Defektfüllmaterial vermieden werden. Teilweise können hiermit der plastische Prozess, d.h. die Umbildung von Fibroblasten und Chondrozyten in dem Recruitment der EPO-Gabe eine Rolle spielen. Therapeutisch wird EPO eingesetzt in der Regeneration von Knorpelstrukturen, wie z.B. hyaliner Knorpel im Bereich der Gelenkflächen für die Kehlkopfenden, Luftröhre, Bronchien und einen Teil des Nasenskelettes. Erfindungsgemäß fördert EPO die Bildung der Knorpelkapseln und die Ausbildung der Proteoglycane / Kollagene. Erfindungsgemäß ist eine Verbindung mit Stammzellen möglich. Aber auch isoliert wird EPO eingesetzt, um elastischen Knorpel zu regenerieren, der z.B. im Kehldeckel-Knorpelbereich lokalisiert ist. Hierzu zählen auch die Knorpel des äußeren Ohres und der Ohrtrompete. EPO wird erfindungsgemäß eingesetzt, um auch Faserknorpel im Verbindungsbereich von Gelenken wiederherzustellen. Hierzu zählen die Regenerationsprozesse im Bereich der Zwischenwirbelscheiben, intraartikulären Fortsätzen, Scheiben und Menisken. Erfindungsgemäß können im adulten Organismus die ansonsten verlorenen regenerativen Veränderungen des Knorpels wieder aktiviert werden, die Regeneration, der Neuaufbau und die Proteoglycan / Collagensynthese der Chondrozyten wieder beginnen. Die Verkalkung des Knorpels kann an den nicht erwünschten Stellen verhindert werden.

Erfindungsgemäß führt EPO in therapeutischen Bereichen zur koordinierten 3-D Regeneration von Knochengewebe als Komplettstruktur inkl. der Gefäße. Bei größeren Defekten können Ersatzstrukturen anorganischer oder biologischer Trägermaterialien eingeführt werden. Hier kann eine Kombination zwischen phasenreinem Tricalziumphosphat und einer EPO-Beschichtung durchgeführt werden.

Alternativ können Hydroxyl-Appatite oder auch biologischer Knochen allogenen oder xenogenen Ursprungs nach entsprechender Behandlung entsprechend eingesetzt werden. Erfindungsgemäß wirkt die Gabe von EPO, auf das Periost und Endost und führt zu einem Recruitment und Vermehrung der dort befindlichen Progenitoren. Danach kommt es zur Ausbildung von Lamellen, Ausbildung von Osteozyten und der entsprechenden Lakunen und Kanalikuli. Erfindungsgemäß wird EPO zur Knochenregeneration eingesetzt, und es kommt zur Ausbildung der Haverskanäle, Knochenkanälchen der Schaltlammellen, der Lakunen, konzentrische Lamellen, die eine benachbarte Lamellensystemabgrenzung und Verbindung ermöglichen. Überraschenderweise werden Volkmann-Kanäle als knöcherne Strassen für die Blutgefäße gefunden. EPO stimuliert die Osteoprogenitoren-Induktion, Osteoblasten, Osteozyten und Osteoklasten. EPO führt bei Osteoblasten zu einer Neosynthese und Induktion der Interzellulär-Substanzen des Knochens. Die Gewebebildung beinhaltet die Ausbildung von knochenspezifischen Zellen. EPO fördert die Ausbildung von Ossifikationskanälen und die Ausbildung von Knochenbälkchen und die Integration vaskulärer Bindegewebsstrukturen. Ausgehend von Verletzungen, z.B. Unfällen, Trauma aber auch Entzündungsreaktionen, kann EPO die Koordinationsaufgaben in der Wiederherstellung der Gewebestrukturen übernehmen, so dass Zonen von Knorpelabbau, Knochenneubildung mit Zonen der Knorpelverkalkung, Zonen der Chondrozytenhypertrophie, Teilungszonen (Säulen, Knorpel und Reserveknorpelzonen (hoher Knorpel)) entstehen.

Erfindungsgemäß fördert EPO die Vaskulogenese auch im Bereich des Knochens, insbesondere die Ausbildung einer Arteria Nutricia, die Metaphysengefäße und die Epiphysengefäße. Zusätzlich werden die Periostgefäße ortsspezifisch integriert.

EPO fördert die Ausbildung des Nukleus pulposus und Anulus pulposus sowie des hyalinen Knorpels im Bereich der Zwischenwirbelscheiben. In Gelenkstrukturen werden Außenligamente, Innenligamente und Kapselverstärkungsbände in der Ausbildung gefördert. Reparaturprozesse der Synovialmembran sind hierbei beinhaltet. Erfindungsgemäß fördert EPO nach Entzündungsreaktionen die Wiederausbildung der glatten Muskulatur, der gestrafften Muskulatur und des Myokardgewebes.

### 7. Glatte Muskulatur

Die Histogenese der glatten Muskelzellen wird durch EPO in der Ausbildung und Differenzierung zu Myoblasten aus Mesenchymzellen gefördert. In der Skelettmuskulatur fördert EPO die Ausbildung des Perimysium und des Endomysium. Durch EPO kommt es zu einer Integration und Koordination des Einwachsens motorischer Nervenfasem und der Ausbildung motorischer Endplatten. EPO fördert die Histogenese des Herzmuskels. Die Herzmuskulatur besitzt normalerweise keine oder nur sehr geringe Regenerationsfähigkeit. EPO wird erfindungsgemäß eingesetzt um die Geweberegeneration der glatten Muskelzellstrukturen, der Skelettmuskulatur und des Herzmuskelgewebes zu erreichen und ein imunmodulatorischen Abkling-Effekt durch Rückkopplung zu erreichen. Dies ist von Bedeutung für die Therapie bei entzündlichen autoimmunen Muskelerkrankungen.

### 8. Nervengewebe

In der dreidimensionalen Geweberegeneration spielt die Verbindung mit Nervensystemen und Innovationsprozessen eine große Rolle. Erfindungsgemäß wird EPO zu einer Koordination der Vaskulogenese mit der Neurogenese und der Zusammenführung dieser Strukturen in der Innovation von parenchymen Drüsen, Geweben und Organen verwendet. Hierdurch kommt es zur Wiederausbildung von synaptischen Kontakten, der Ausbildung von Neuronen als Laminä und vertikalen Einheiten und der Ausbildung von Nervenfasern und Fasertrakten. Verbindungsstrukturen im Bereich der Ganglienplexen werden hervorgerufen. Die Hirnnerven und Kranialnerven werden einbezogen, die Spinalnerven werden nach Trennungen regeneriert und sowohl efferente motorische als auch afferente oder sensible Strukturen werden wieder neu geschaltet. Unterstützend können Nervenleitschienen eingesetzt werden, die aus Biopolymeren, (biodegradable oder nicht-biodegradable (Silikon, Polyurethan)) bestehen können oder biologischen Ursprungs, oder synthetischen Ursprungs (z.B. Collagen, Elastine, Fibronektine, Polylaktid, Polyhydroxybutyrate, Seide) sein können und in Kombination mit Stammzellen verwendet werden können. Die Stammzellen können mesenchymalen Ursprungs sein. Sie können aber auch aus den Gewebestrukturen der neuronalen Strukturen direkt rekrutiert werden, wobei es sich nicht um eine Apoptosevermeidung handelt, sondern um eine Initiation des Neuritenwachstums und der Vermehrung in eine gerichtete dreidimensionale Struktur.

Ein wichtiger Effekt der EPO-Wirkung ist die Geweberegeneration im Bereich der Bindegewebsstrukturen des zentralen Nervensystems, insbesondere der Meningen und deren begleitenden Blutgefäße. Diese Wirkung ist sehr wichtig für die Regeneration von Ganglien und Sinnesorganen. Erfindungsgemäß wird die Ausbildung von Axonstrukturen und die Ausbildung von Axonen ermöglicht. Die elektrophysiologische Aktivität und der axonale Transport wird wieder hergestellt, sobald eine Quervernetzung erreicht wird. Durch kombinierte Gabe mit Vitaminen, zum Beispiel Vitamin C, können diese Prozesse unterstützt werden. Wichtig aber ist die erfindungsgemäße Wirkung in der Gewebsregeneration in dem Zusammenspiel zwischen einer Aktivierung durch ortsständige Plasmazellen, Makrophagen, Lymphozyten unter Umständen Mastzellen, die zu einem zellulären Rekruitment und einem reparativen Verhalten führen (nach therapeutischer EPO-Gabe). Im zentralen Nervensystem kann EPO erfindungsgemäß auch für die Regeneration im Bereich degenerierter Strukturen eingesetzt werden. Hierzu zählen Morbus Parkinson und Degeneration im Striatumbereich sowie Morbus Alzheimer und die amyotrophe Lateralsklerose, die auch in die Peripherie ausstrahlt sowie die Multiple Sklerose und bei organisch bedingten Depressionen. Wichtig hierbei ist die Behandlung mit Entzündungsreizen, so dass die Aktivierungskaskade, die durch EPO ermittelt wird, initiiert werden kann. Eine stereotaktische Implantation mit mesenchymalen Progenitoren und neuronalen Vorläuferzellen wie auch die Verwendung ortsständiger Progenitonen regionaler und peripherer Monozyten kann durch EPO therapeutisch verstärkt und zur Regeneration geführt werden. Das Ziel ist die Ausbildung neuer neuronaler Zellen des Zentralnervensystems. Erfindungsgemäß kommt es danach zu einer Funktionsaufnahme der Zellen mit einer Bildung neuroaktiver Peptide und Endorphinen sowie z.B. Serotonin, Dopamin, Noradrenalin, Actylcholin. Synaptische Prozesse werden wieder verbunden. In Begleitfunktionen der Regeneration werden auch nicht die neuronalen Zellen des ZNS, wie zum Beispiel die protoplasmatischen Astrozyten, der fibrilläre Astrozyt, Oligodendrozyten, Mikrolia (Glioblasten) und das Ependym miteinbezogen. Das Nervengewebe wird durch EPO nicht nur in einer neuronalen Regeneration, sondern auch in der begleitenden Stützzellstruktur der sogenannten Neuroglia gebildet. In der Proportionalität übertreffen die Neurogliazellen die neuronalen Zellen bis zum Faktor 10. Sie bilden das Bindegewebsstrukturgerüst des ZNS und führen die Blutgefäße. Dies hat eine wichtige regenerative Komponente für den strukturellen Wiederaufbau. Als Ependym versteht man die Epithelauskleidung der inneren Oberflächen der Ventrikel im ZNS.

EPO führt erfindungsgemäß zu einer Regeneration der Axonenhüllen, einer sogenannten Schwannschenscheide. EPO fördert die Ausbildung der Myolinscheide (Markscheide). Im ZNS liefern Oligodendrozyten die Myolinscheiden. Dies ist therapeutisch besonders wichtig bei Erkrankungen der Markscheidenausbildung. Bei den peripheren Nerven werden die Begleitzellen induziert, Ganglien zu umkleiden. Die Schwannzellen werden durch EPO stimuliert im Bereich des Regenerationsprozesses die neue Ausbildung der peripheren Nerven mit zu ermöglichen. Es kommt hier zur Ausbildung der interperiodischen Linien der Myelinsegmente.

EPO fördert erfindungsgemäß die Ausbildung der grauen und weißen Substanz und deren gewebespezifische Differenzierung im ZNS. EPO unterstützt die Ausbildung von Ganglien (Ansammlung von Nervenzellen außerhalb des ZNS). Nach traumatischen Situationen kommt es zur Regeneration dieser Strukturen. Die Regenerationsprozesse auch im regenerativen Bereich werden über die EPO-Modulation zu einer Neoinnervation geführt, wobei es ausgehend von Tränendrüse, Unterkieferdrüse, Unterzungendrüse sowie Ohrspeicheldrüse, Herz, Kehlkopf, Luftröhre, Bronchien, Lunge, Magen, Dünndarm, Blutgefäße des Bauchraumes und weitere Blutgefäße, Leber, Gallenblase, Gallengänge, Bauchspeicheldrüse, Nebennierenmark, Dickdarm, Mastdarm, Niere, Harnblase und Geschlechtsorgane zu einer Neoinnervation kommt als reparativer Prozess. Im Bereich des sympathischen Grenzstranges und des sympatischen Gangliums (Ramus communicans albus und ramuns communicans griseus und nervus splancnicus, Ganglium coeliacum und ganglium mesintericum superius) kommt es zu reparativen Prozessen. Die Nervenendungen können durch die Regenerationsprozesse auch in die mehrschichtigen Plattenepithelstrukturen integriert werden.

Histologisch werden Endkolbenstrukturen, Körperchen und sogenannte Pacini-Körperchen an den sensiblen Strukturen wiederhergestellt. EPO unterstützt die Ausbildung der Dura mater, deren Struktur wiederum Bindegewebsleitstrukturen sensibler Nervenblutgefäße enthält. Dadurch kommt es zur Ausbildung Subduralraumes (Ausbildung des duramatis spinalis) und des Epiduralraumes. Die dura mater encephalica haftet am dem Schädelknochens an und wird durch die EPO-Stimulation im Rahmen des Regenerationsprozesses wieder aufgebaut. Die Arachnoidia erstreckt sich in den Subarachnoidialraum. EPO-Regenerationsprozesse stimulieren auch die Wiederherstellung des Subarachnoidialraums und der weichen Himhaut. Diese kann Beziehungen zum kardiovaskulären (Virchow-Raum) aufnehmen. EPO führt zu einer Wiederherstellung der dünnen Bindegewebslage die die Oberfläche von Gehirn und Rückenmark bedeckt. Es kommt zur Regeneration der venösen Sinus der Arachnoidiazotten. Insbesondere der plexus chorioidius und die Hirnventrikel werden regeneriert. Die erfindungsgemäße Gabe von EPO führt bei Entzündungsprozessen zu einer Wiederausbildung der Bluthimschranke durch strukturelle Geweberegeneration. Die funktionelle Bedeutung dieser Regeneration die sich über das gesamte Himgewebe einschließlich der medulla oblongata mit Ausnahme der Neurohypophyse erstreckt, ist von großer Bedeutung für die Homöostase des ZNS. Die Barriere wird mit Astrozyten durch EPO-Gabe wieder erneut ausgebildet.

Histogenese des Nervensystems durch EPO-Einwirkung

Nach Trauma im zentralen Nervenbereich und degenerativen Prozesse sind das Zusammenspiel zwischen Gliazellen, Oligodendrozyten, Neuronen, Subduralraum und Blutgefäßen gestört. Diese räumlichen Verbindungen und die darin befindlichen Begleitzellen sind in einer Histogenese physiologisch beteiligt. Dennoch kommt es bei Verletzung zu Heilungsproblemen, da die neuronalen adulten Gewebe nicht regenerationsfähig im gewünschten Umfang sind. Durch die Gabe von EPO kann erfindungsgemäß dieser Prozess in eine regenerative Richtung beschleunigt geführt werden und die strukturelle Wiederherstellung durch die Verbindung der strukturellen Anteile mit den Parenchymanteilen erreicht werden. In der Embryonalentwicklung läuft dieser Entwicklungsschritt nach einem festgesetzten Zeitplan ab. EPO ist im ZNS eine vermittelnde Struktur in Verbindung mit z.B. dem Nervenwachstumsfaktor (NGF). Die mangelnde Regenerationsfähigkeit des ZNS im adulten Organismus wird durch EPO-Gabe dadurch kompensiert, dass ein Recruitment der ortsständigen Zellen, immunmodulierende Zellen, Abräumzellen (Makrophagen) und Stammzellen erfolgen kann. Stammzellen (z.B. autolog aus peripheren Blut CD45 positiven Zellen, mesenchymale Stammzellen, aus Fettgewebe, aus Nabelschnurgewebe) und andere Stammzellen können kombiniert werden. Die Verwendung autologer Gewebe ist der Verwendung allogener Zellen (z.B. embryonaler Zellen) vorzuziehen, von dem EPO Effekt aus immunologischen Gründen abzutrennen aber nicht auszuschliessen).

In Kombination mit stereotaktischen Operationen oder Infusionen können die Progenitoren ortsspezifisch positioniert werden. Durchtrennte oder verletzte periphere Nervenfasern können durch EPO-Gabe in Verbindung mit Leitstrukturen, Leitschienen oder auch in Verbindung mit biologischen Strukturen vor Ort (aus der unmittelbaren Umgebung) in die gewünschten Bereich gelenkt werden z.b. auch durch Fibrinstrukturen. Fibroblastenstrukturen können auch in anderen Bereichen mit Stammzellen und EPO integriert werden. Alternativ können auch biologische Matrixmoleküle auf der Basis von Kollagenen oder Elastinen in rekombinanter Form oder bestehend aus Peptidsequenzen, in Gemische eingebracht werden, wodurch es zu Injektionslösungen kommt, die durch EPO-Gabe vermittelt werden können. Parallel kann die Neuregeneration auch systemisch unterstützt werden durch Gabe von EPO. Ohne die Gabe von EPO und die Stammzellunterstützung kommt es zur sogenannten Wallerschen Degeneration, die seit 1850 bekannt ist. Entlang des sogenannten Büngner Bandes kommt es zu Regenerationsprozessen. Im ZNS kommt es nach 12 bis 24 Stunden nach einer Läsion zu terminalen Degenerationsphänomenen. Der Zellkörper löst sich durch Chromatolyse auf. Die regenerativen Prozesse, die durch EPO beschleunigt werden, führen zu einem Axonenaufbau. Durch EPO wird die Nesselsubstanz und der Golgi-Apparat erneuert. Die Schwellungen des Zellkörpers nehmen ab, während in physiologischer Weise in der ersten Woche nach einer Läsion Regenerationsphänomene bei peripheren Axonen auftreten. Durch die erfindungsgemäße Gabe von EPO kommt es zu Beschleunigungen der Regeneration, sodass unterhalb einer Woche bereits Regenerationsphänomene, wie die Ausbildung von Philopodien und protoplasmatischen Wachstumskeulen ermöglicht werden. Die axonalen Philopodien benötigen normalerweise zwei Wochen, um in den Schwannzellen Höhlen in das distale Segment einzuwachsen. Durch biologische Strukturen und Kollagene können diese Strukturen bei Komplettdurchtrennung überbrückt werden. Eine Anwendungsform für die Nervenschienung stellen z.B. EPO beschichtete Kollagenröhrchen dar. Andere Trägermaterialien wie Polylaktid bzw. Poldhydroxyalkanoate können ebenso verwendet werden. Die Kombination mit Stammzellen und autolegem oder allogenem Fibrin als Leitstruktur ist möglich.

Bei Polymyolitis-Zuständen wird durch EPO-Gabe eine Neosynthese des Perineuriums erreicht. Dieser fördert die Ausbildung von kollateralen Sprossungen im ZNS.

### 9. Leber

Nach viralen Infektionen der Leber (z.B. Hepatitis A, B, C, D) kann es zu Regenerationsdefekten der Leber kommen. Die Lebercirrhose stellt das Endstadium eines Regenerationsdefektes der Leber dar, bei der es zu bindegewebigem Ersatz der parenchymatösen Zellen gekommen ist, da die körpereigene Regeneration nicht entsprechend mit der Rate einer durch Noxen bedingten Schädigung durch kontinuierliche Regeneration mithalten kann.
Nach Trauma und operativen Eingriffen, z.B. bei Tumorresektionen der Leber, kommt es zu einem akuten Verlust viablen Gewebes und zur Gewebezerstörung.

Erfindungsgemäß kommt es nach Gabe von EPO zur beschleunigten Regeneration der Hepatozyten und nicht-parenchymalen Zellen der Leber bis zur Restitutio ad Integrum. Charakteristisch-hierfür ist die Wiederausbildung einer normalen Leberläppchen Architektur, die Wiederausbildung des interlobulären Bindegewebes, der Zentralvenen und der Glisson-Trias: Dort werden erkennbar die Gallengänge, Lymphgefäße, periportale Bindgewebe, Arterien und Vena interlobularis neben den Leberzellen wiederhergestellt.
Die spezifische Ausbildung der intralobulären Retikulinfasergeflechts und der Lebersinusoide wird wieder hergestellt. Der histologische Aufbau nach Regeneration zeigt die typische Balkenstruktur. Die Fensterungen im Bereich der Endothelzellen weisen auf eine typische Leberausprägung hin. Wichtige Initiatorzellen nach Trauma sind die Kupfferzellen die IL-6, IL-1, TNF-alpha freisetzen.

Die Gabe von EPO führt zu deutlich höheren Regenerationsprozessen selbst im Vergleich zu Lebergewebe ontogenetisch junger Lebern. In vitro-Faktorerhöhung um Faktor 10 bis 30 werden erreicht.

### 10. Gallenblase:

Bei Entzündungen der Gallenblase und auch nach mechanisch bedingten Verletzungen der Gallenblase kommt es bei therapeutischer EPO Gabe zu einer Wiederherstellung des normalen Wandaufbaues wie folgt: Bindegewebe (Kapsel, mit Leberkapsel verwachsen), Tunica muscularis, Luschka Gänge, Lamina propria (reich vaskularisiert) und Tunica mucosa (mit Epithel, einschichtig, hochprismatisch). Lymphgefäße, Nerven aus dem Bereich des Vagus werden wieder angeschlossen.

### 11. Pankreasregeneration:

Die EPO Gabe unterstützt eine Regeneration der B-Zellen, D-Zellen und A-Zellen des Pankreas. Die Regeneration beginnt aus den kleinen Epithelsträngen, die aus den Ausführungsgängen hervorgehen. Die Venen und Arterien des Pankreas werden mitregeneriert.
Die regenerativen Prozesse betreffen den endokrinen und exokrinen Anteil nach Trauma, entzündlichen Erkrankungen oder immunologischen Automimmunprozessen.

### 12. Nasenhöhle und Nasennebenhöhlen:

Septum nasi, Vestibulum nasi, Nasenhöhle, Nasenmuscheln unterliegen nach Verletzungen und EPO Gabe regenerativen Prozessen. Das Epithel wird wiederhergestellt und ebenson die lamina propria (straffes, kollagenes Bindegewebe mit tubuloalveolären mukoserösen Drüsen), Gefäße mit arteriovenösen Anastomosen und Schwellkörpern, adrenerge und cholinerge Nerven. Im Bereich der Riechschleimhaut wird das sehr hohe Epithle und die serösen Bowman-Drüsen wiederaufgebaut.

### 13. Kehlkopfbereich (Larynx):

Das mehrreihige hochprismatische Flimmerepithel, die lamina propria, die serösen Drüsen und die gemischt ― seromukösen Drüsen werden durch EPO / TPO Gabe mit und ohne Stammzellkombination wieder hergestellt.

### 14. Luftröhre:

Wiederherstellung des typischen Wandaufbaues mit Perichondrium, hyaliner Knorpel, Perichondrium, lamina propria mit seromukösen Drüsen, Submukosa, Lamina propria und Epithel. Die Adventitia wird an der Peripherie regeneriert und die Knorpelspangen durch glatte Muskulatur verbunden. Im Eptihel befinden sich Becherzellen, Basalzellen und Flimmerzellen. Ebenso endokrine (Pa-) Zellen, Typ I und Typ II Bürstenzellen.

### 15. Lungen:

Die komplexe 3-D Architektur und Struktur der Lunge wird erfindungsgemäß wie folgt wiederhergestellt. Arteria bronchialis, Drüsen, Bronchus, Äste der arteria pulmonallis, Bronchiolen, glatte Muskulatur und Äste der vena pulmonalis, ducti alveolari, Alveolensäckchen und die Pleura-Umkleidung wird dreidimensional zusammengeführt. In den bronchopulmonären Segementen werden intersegementale Septen, Segementvenen und Segmentarterien regeneriert. In den Bronchien werden Epithel, lamina propria, tunica muscularis, Drüsen und Knorpel regeneriert. In den Alveolen wird die Blut-Luft Schranken-Funktion wiederhergestellt. Elastinstrukturen werden regeneriert. Alveolarepithelzellen (Typ I und II) treten wieder auf. Blutgefäße, Lymphgefäße und Nerven (plexus pulmonalis) werden dreidimensional koordiniert duch EPO und / oder Stammzellintegration eingefügt.

### 16. Harnsystem:

Die regenerativen Wirkungen im Bereich der Niere blieben bisher unbekannt, da EPO bei chronisch insuffizienten Nieren substitutiv für den EPO Mangel eingestetzt wurde. Bei traumatischen Verletzungen, Entzündungen und toxischen oder immunologischen Schädigungen kommt es erfindungsgemäß durch EPO Gabe mit und ohne Stammzellen / Progenitoren zur Regeneration der urologischen Systeme. In der Niere kommt es zur Regeneration der Rindenstruktur, der Nierenkelche, des Marks, der Markstrahlen, Nierenpyramiden und der Columna renalis.

Es kommt zur Regneration der Mesangialmatrix, der Mesangiumzellen, des Glomerulus (mit lamina rara intema, lamina densa, lamina rara externa. Die strukturellen Beziehungen der Kollagenfilamentbündel, der Mesangialzellen, Podozyten und Endothelzellen werden wieder hergestellt. In der Nierenrinde werden Sammelrohr, Hauptstück, Mittelstück, Macula densa, juxtaglomuläre Zellen und Vas afferens regeneriert. Im Mark der Niere kommt es zur Wiederausbildung der charakterisistische interstitiellen Zellen und des ductus papillaris.

Die Harnleinterstruktur wird in die Tunica adventitia, Ringmuskulatur und Längsmuskulatur der Tunica muscularis, lamina propria, und Epithel (tunica mucosa) regeneriert. Die Harnblase wird zur Wiederherstellung des Epithels, lamina propria (tunica mucosa), tunica submucosa (Längs- und Ringmuskulatur) und äußere Längsmuskulatur geführt.

Die weibliche Harnröhre wird mittels EPO und / oder Stammzellen zur Regeneration der Tunica muscularis, des venösen Geflechtes, der Drüsenlakunen, der lamina propra und des überwiegend unverhomten Plattenenepithels geführt. Bei der männlichen Harnröhre wird die pars prostatica, der musculus sphincter uretrhae und das diaphragma urogenitale, utriculus prostaticus, pars membranacea, pars spongiosa und die fossa navicularis regeneriert. Auf die Schleimhaut der pars spongiosa folgt das corpus spongiosum. Nach traumatischen Verletzugnen oder operativen Eingriffen kann EPO in Verbindung mit Fibrinkleber mit und ohne Stammzellen in die strukturellen Defekte verbindend injeziert werden oder entsprechend topisch aufgebracht werden.

### 17. Endokrine Drüsen:

Im Bereich der Hypophyse, Epiphyse, Schilddrüse, Nebenschilddrüse, Bauchschpeicheldrüse (Langerhansinseln), Nebennieren, Ovarien und Hoden kann es durch traumatische Verletzungen, Operationen (z.B. Tumorresektionen) zu Gewebeverletzungen kommen. In diesen Fällen kann EPO erfindungsgemäß in Verbindung mit Stammzellen aus dem autologen Knochenmark, Nabelschnurstammzellen, Progenitoren aus dem peripheren Blut (Monozyten) oder durch Recrutiering ortständiger Progenitoren zu einer Geweberegeneration führen bzw. diese unterstützen. In der Adenohypophyse werden die basophilen Zellen, die arteriae hypophysales superiores und inferiores, die gomitoli sowie die Portalgefäße wiederhergestellt. Die Neurohypophyse wird in ihrer Zusammensetzung mit Pituizyten, marklosen Nervenfasern aus neurosyekretorsichen Neuronen im Hypothalamus regeneriert. Hierzu werden erfindungsgemäß EPO ― Fibrinstränge als Leitstrukturen mit Stammzellen zwischen den Regionen gelegt. Kapselstrukturen werden wie auch in anderen Himregionen regeneriert. Bei der Epiphyse wird Habenula, Recessus pinealis, Läppchenstrukturen, Himsand, Bindegewebssepten und Pia mater (Kapsel) wieder hergestellt. Nervenfasern, Pinealozyten, Astreozyten werden in die spezifischen Strukturen der Epiphyse verbunden.
Die Schilddrüse unterliegt häufigen operativen Eingriffen. Die Follikelepithelzellen, C-Zellen, Bindgewebsstrukturen werden entsprechend regeneriert. Nebennieren bestehen nach der erfindungsgemäßen Regeneration aus Kapsel, zona glomerulosa, zona fasciculata, zona reticularis. Spongiozyten befinden sich in der zona fasciculata.

### 18. Fortpflanzungssystem:

Regenerative Reparaturprozesse können erfindungsgmäß durch strukturelle Prozesse bei Ovarien zu einer Wiederherstellung des funktionellen Aufbaues führen. Hierzu sind insbesondere die Regenerationsprozesses des Stroma ovarii, der tunica albuginea, die granulosa Zellen, theca folluculi intema und extema involviert. Die Mesothelauskleidung wird nach Verletzungen wieder geschlossen. In den Eileitem wird der Wandaufbau wiederhergestellt (Epithel, lamina propria (Schleimhaut)), Ringsmuskulatur, Längsmuskulatur (Muskelschicht), subseröses Bindegewebe, Mesothel (Serosa)).

In der Ampulle des Eileiters werden Drüsenzellen, lamina propria und Flimmerzellen regeneriert. Corpus uteri wird in Endometrium, Myometrium und Perimetrium strukturell regeneriert. Schleimhaut (Epithel, lamina propria), Muskelschicht (Längsmuskulatur, musculus bulbospongiosus) und Adventitia der Vagina werden regeneriert. Lymphgefäße und Nervenversorgung werden wiederhergestellt. Neben dem gesamten Gewebebereich der weiblichen Geschlechtsorgane sind ebenso bei den männlichen Geschechtsorganen Reparaturprozesse nach Trauma und sonstigen Verletzungen möglich. So werden u.a. Hoden, Nebenhoden, Penis (inkl. corpus cavernosum), Ductus seminiferus, Prostata und deren strukturelle Einbindung in das umgebende Weichund Hartgewebe koordiniert regenerierbar. Die blutversorgenden Strukturen, Lymphgefäße und Nerven werden reintegriert.

### 19. Milchdrüse:

Der strukturierte Aufbau in Läppchen, Bindegewebe, Milchsäckchen, Ausführungsgänge mit dem histologischen Detailaufbau Alveolen, intra- und interlobuläres Bindegewebe, Milchgang, Myoepithelzellen findet durch Regeneration wieder statt. Insbesondere nach Tumorresektionen oder plastischen Eingriffen kann das Brustrdüsengewebe in Verbindung mit biologisch modellierbaren scaffolds, EP und Stammzellen neu aufgebaut werden.

### 20. Zentrales Nervensystem:

Im Gehirn und Rückenmarkbereich spielen insbesondere mechanische und ischämische Insulte mit nachfolgender Gewebedegeneration bzw. Strukturunterbrechungen eine klinisch bedeutende Rolle. Graue und weiße Substanz, Kleinhirn, Mittelhirn und Grosshirn, Kerngebiete und Faserbahnen werden strukturell zusammenführt und regeneriert.
Zum Zwischenhirn zählt der Epithalamus, Thalamus dorsalis, Subthalamus und Hypothalamus. Der Bereich des Endhims umfasst zytoarchitektonsich ca. 50 Felder die strukturell regeneriert werden können. Die Hauptgebiete sind Frontallappen, Temporallappen, Parietallappen und Okzipitallappen. Die Septumregionen, der Bulbus olfactorius sowie die Cortexbereiche können regeneriert werden. Zu dem Regenerationsbereich der Basalganglien gehören das Corpus striatum mit Nucleus caudatus und Putamen, das ventrale Striatum, sowie nucleus accumbens und der nucleus basalis. Durch die Regenerationsprozesse können die Ausfälle motorischer Areale mit den dazugehörigen Lähmungen und die Ausfälle sensorischer Neurone revidiert werden. Die Schädigungen im Bereich der motorischen Rinde (z.B. durch Geburtstraumata und Unfälle) mit den daraus resultierenden spastischen Lähmungen können revidiert werden. Bei den Schädigungen der Vorderhome mit den einhergehnden schlaffen Lähmungen können ebenso regenerative Prozesse induziert werden. Störungen der Hippocampusfunktionen rufen schwere Störungen im Bereich der Orientierungs- und Merkfähigkeit hervor (Korsakoffsyndrom). Bei Schädigungen kortikaler Neurone kann die damit einhergehende Gefahr der Alzheimerentstehung eingedämmt werden. Bei zerebrovaskulärer Insuffizienz bis zum Himinfarkt können reparative Prozesse durch Regeneration eingeleitet werden.

### 21. Auge:

Die Regenerationsprozesse erstrecken sich auf die Hornhaut, Bindehaut, Regenbogenhaut, Schlemmkanal, Ziliarmuskel, Linse, Zonulafasern, Ora serrata, musculus rectus medialis, Glaskörper, Retina, Gefäße, dura mater, arachnoidea, Sehnerv, arteria und vena centralis retinae, Netzhaut, Aderhaut und Lederhaut. Der spezifische Aufbau der Cornea mit Kollagenlamelle, Fibroblasten, Elastinfaseren, Kollagenlamelle, Bowmann Membran, Epithelschicht wird wiederhergestellt. Die Sklera (Lederhaut) wird in lamina fusca, substantia propria und Episklera regeneriert. Linsenepithelien, Augenlider mit epidermalen und dermalen Anteilen, Unterhaut, Schweissdrüsen, Haarfollikeln, Epithel, lamina propria, Tarsus, Melborn-Drüse, Moll-Drüse, musculus levator palpebrae, musculus orbicularis oculi wird wiederhergestellt.

### 22. Ohr:

Ohrmuschel, Mittelohr, Trommelfell, Paukenhöhle, Gehörknöchelchen, Musculus stapedius, musculus tensor tympani, antrum mastoideum, cellulae mastoideae und Ohrtrompete, Innenohr und knöchernes Labyrinth, Cochlea mit den dazugehörigen Nerven, Labyrint und entsprechend das Corti ― Organ sind erfindungsgemäß in die regenerative Prozesse miteinbezogen.

### II. Ausführungsbeispiele

### 1. Zelltransplantation

### Hepatozyten:

Leberzellen werden durch Collagenase-Verdau in üblicher Weise aus nicht tranplantierbaren Organen bzw. Leberresektaten isoliert. (Bader, A., Rinkes, I.H.B., Closs, I.E., Ryan, C.M., Toner, M., Cunningham, J.M., Tompkins, G.R., Yarmush, M.L. (1992) A stable long-term hepatocyte culture system for studies of physiologic processes: Cytokine stimulation of the acute phase response in rat and human hepatocytes. Biotechnol Prog. 8, 219-225.)

Die isolierten bzw. vorkultivierten Zellen werden in flüssigem Stickstoff gelagert. Nach dem Auftauen der Zellen entsprechend bekannter Protokolle (Karim, N., Allmeling, C., Hengstler, J.-G., Haverich, A., Bader, A. (2000) Diazepam metabolism and albumin secretion of porcine hepatocytes in collagen-sandwich after cryopreservation. Biotechnol Letters 22: 1647-1652) werden die Hepatozyten Suspension / Kulturen mit 100-150 IU/kg/KGW Epoetin alfa (bezogen auf den Empfänger) versetzt. Epoetin alfa wird hierzu in einer sterilen Lösung in einem Volumen von 1 - 1.5 ml einer 10 ml Hepatozytensuspension mit einer Konzentration von 2-10 Millionen / ml Zellen zugegeben.

Diese Suspension wird langsam (1 ml / Minute) intraportal injiziert. Die Suspension kann zusätzlich mit 1000 IE Heparin versetzt werden, um eine Thrombosierung zu vermeiden.

Alternativ kann das Zell / EPO Gemisch auch unter die Leberkapsel oder direkt in das Leberparenchym an mehreren Stellen injiziert werden. Hierzu empfiehlt es sich die Konzentration der Hepatozyten um den Faktor 2-5 zu erhöhen und das injizierte Volumen entsprechend zu reduzieren.

Die Stichkanäle werden mit handelsüblichen Fibrinkleber (Baxter Tissucol) verschlossen. Alternativ kann eine Tamponade mit Kollagenflies verwendet werden. Die Tamponade kann ebenso mit der Epoetin alfa Lösung versetzt werden. Es ist darauf zu achten, dass die Tamponade an der Adhäsionsstelle noch trocken bleibt.

Der Fibrinkleber stellt ein 2 Komponentenmix dar. Üblicherweise besteht eine Komponente aus Fibrinogen und die andere aus einer Aktivierungslösung mit Ca⁺⁺ und Proteinaseinhibitoren (z.B. Aprotinin). In die Aktivierungslösung kann Epoetin alfa durch Hinzumischen auf eine Endkonzentration von 100-150 IU/kg/KGW gegeben werden.

In analoger Weise können Stammzellen aus dem Knochenmark, dem Fettgewebe, einem spezifischen Parenchym oder dem Blut (Aufreinigung aus Buffy Coats, CD 34 positive Zellen) aus Nabelschnurblut und den mesenchymalen Zellen aus dem Nableschnurgewebe verwendet werden.

Parallel zur Zelltransplantation in ischämisch, toxisch, infektiös oder mechanisch (traumatisch) geschädigte Bereiche können die Zellen in einen Fibrinkleber oder autologes Plasma eingebracht und unter Zusatz von Epoetin alfa (100-150 IU/kg/KGW) zur Polymerisation gebracht werden.

Parallel dazu kann eine EPO-Gabe von jeweils 10000 IE s.c. begonnen werden, so dass insgesamt 40000 IE innerhalb einer Woche appliziert werden.

Das Ergebnis ist eine um den Faktor 2-3 erhöhte Geweberegeneration, die zu einer strukturellen Reparatur führt.

### 2. Postoperative Gabe

Nach ausgedehnten Operationen im Herz-Thorax-Gefäßbereich, der Visceralchirurgie, der Plastischen Chirurgie aber auch nach ausgedehntem Trauma entstehen Defektsituationen, die zu einer vitalen Gefährdung des Patienten führen können.

Epoetin alfa wird hier in einer Konzentration von 100-150 IU/kg/KGW gegeben.

Durch die EPO Gabe kommt es zu einer endogenen Erhöhung des Wachstumshormons um den Faktor 2, wodurch die Geweberegeneration nach der Operation beschleunigt wird. Die restitutio ad integrum tritt um ca. 1-2 Wochen früher als bei vergleichsweise ohne EPO Gabe behandelten Patienten ein.

EPO kann auch nach einer split liver Transplantation bzw. Lebertranplantation zur Induzierung der Leberregeneration gegeben werden.

Nach einer Lebertransplantation kommt es vor, dass das transplantierte Gewebe - oder bei Lebendspendem - das verbleibende Gewebe nicht rasch genug und in ausreichender Masse als aktives Gewebe zur Verfügung steht. In diesem Fall kann bereits 24 h vor der Operation und zum Zeitpunkt der Operation und danach in 24 h Abständen 100-150 IU/kg/KGW Epoetin alfa gegeben werden. Hierdurch kommt es zu einer deutlich beschleunigten Leberregeneration wobei postoperativ insbesondere am 4-5 Tage eine Volumenzunahme im Ultraschall diagnostizierbar ist.

### 3. Gabe nach operativer Leberresektion bei gut - und bösartigen Tumoren

Bei einer ausgedehnten Leberresektion besteht der Bedarf, eine beschleunigte Regeneration zu erreichen, da die Verfügbarkeit einer ausreichenden Zellmasse wichtig für das Überleben der Patienten ist. Nach operativer Resektion kann bei systemischer Gabe 100-150 IU/kg/KGW Epoetin alfa oder einer entsprechenden Menge bei topischer Applikation (in Fibrinkleber, Plasma) gegeben werden.

Bei Verdacht auf persistierenden Tumorbefall bzw. nicht ressezierbaren Tumormetastasten können sowohl systemisch als auch topisch entsprechend dem Tumortyp und der Prognose übliche Cytostatika in Kombination mit EPO verabreicht werden.

Nach Resektion und Gabe von EPO kommt es zu einem beschleunigten Grössenwachstum der behandelten Gruppe um 30% im Verhältnis zu einer nichtbehandelten Gruppe. Vor allem die Hepatozyten an den Resektionsflächen treten verstärkt in den strukturellen Wachstumsprozess ein. Es kommt zu einer vollständigen Ausbildung des Gefäßbaumes und des darum befindlichen Gewebes. Hierbei sind die Hepatozyten in typischer dem normalen Organ entsprechender Weise in Lobuli mit Gefäßversorgung, Zellplatten mit nichtparenchymalen Zellen (Kupffer, Pit, Ito - und Endothelzellen) angeordnet.

Es kommt zu einem systemischen Grössenwachstum. Das Größenwachstum endet bei Erreichen der initialen Grösse.

Die Gruppe mit perioperativer EPO-Gabe weist bereits am 1-2 Tag postoperativ einen um etwa 0.5 g/dl höheren Hb-Wert auf. Dies ist als Zeichen der bekannten Wirkung des EPO zu werten. Parallel kommt es jedoch zur Leberregneration. Hier vermehren sich nicht nur die Hepatozyten sondern alle Zelltypen und vor allem auch die Bindegewebsstrukturen, die das Architekturgerüst der Leber darstellen.

### Versuchsdurchführung:

28 weibliche Schweine (Gewicht 40,0 - 62,0 kg) wurden nach dem Zufallsprinzip in drei Gruppen aufgeteilt. Die Teilentfernung der linksseitigen Leber wurde mit der Technik der Bauchendoskopie durchgeführt.

Der Kontgruppe (n=16) wurde kein EPO gegeben. Der Gruppe 2 (n=6) wurde eine Kombination aus 10.000 Einheiten EPO und einem Fibrin-Dichtungsmittel (Quixil) lokal auf die Leberresektionsoberfläche gegeben. Gruppe 3 wurde gleichermaßen behandelt; die Schweine erhielten jedoch zusätzlich zur lokalen EPO Behandlung 10.000 Einheiten EPO systemisch am Tag 0, 3, 7 und 11.

Leberproben wurden von dem herausgeschnittenen Leberstück am Tag 0, 24 Stunden nach Resektion aus einem Bereich 1 cm unterhalb der Resektionsoberfläche und 14 Tage nach Resektion unterhalb der Resektionsoberfläche und des rechten seitlichen Lappens entnommen.

Für den Nachweis von Ki-67 Antigen, PCNA (proliferating cell nuclear antigen = PZNA Proliferation Zellkern Antigen) und Apoptose, wurde der Streptavidin-Biotin Immunperoxidase Assay an mit Formalin fixierten und in Paraffinwachs eingebetteten Lebergewebe durchgeführt.

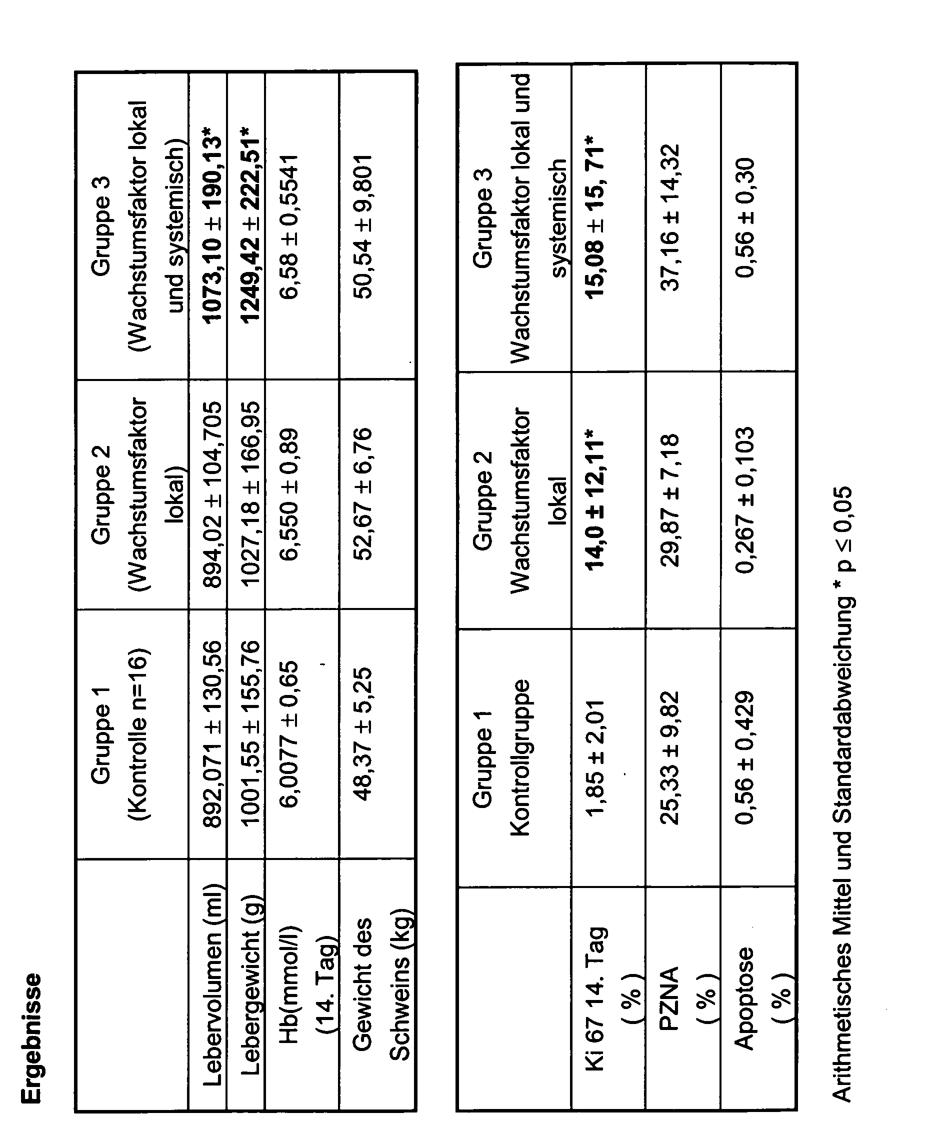

### 4. Optimierte Endoprothese bzw. Implantate

Implantate können aus biologisch abbaubaren aber auch aus permanenten Materialien bestehen. Ein Beispiel hierfür sind metallische Endoprothesen z.B. im Hüftbereich. Im Anschluss an die Herstellung der metallischen Rohform wird eine Mikrostrukturieurng durch Abrasion, Ätzen oder Laserbehandlung erreicht. Hierdurch können offene Porositäten und Rauhigkeiten im Bereich von .1 bis 50-60 µm erzeugt werden.

Diese Strukturen werden anschließend in einer Lösung aus phasenreinem beta Tricalziumphosphat aufgefüllt, so dass eine homogene Oberflächenbeschichtung erreicht wird. Danach werden die Strukturen idealerweise einem weitergehendem Sinterungsprozess unterzogen, um die beta-TCP Strukturen zu festigen.

In den mineralischen Strukturen können lösliche Salze / Zucker eingebaut werden, bzw. Gasbildungen induziert werden um weitere interkonnektierende Porositäten zu erreichen. Im Anschluss an diesen Fertigungsprozess werden die Strukturen mit dem erfindungsgemäßen Wachstumsfaktor oder dessen Derivaten, Teilen oder Analoga imprägniert bzw. beschichtet. Depots können entsprechend an den Oberflächen gesetzt werden, indem die Kavitäten sich mit EPO füllen bzw. slow release Substanzen verwendet werden. Alternativ kann auch unmittelbar vor der Implantation das steril aus der Verpackung entnommen Implantat mit EPO und deren Analoga beschichtet werden.

Es kommt dadurch zu einem verbesserten Gewebeverbindungsprozess mit dem Implantat. Der Knochen wird makrovaskulär verbunden und dass Implantat beschleunigt und nachhaltiger ossär integriert.

In ähnlicher Form können Implantate im Mund-, Kiefer ― und Gesichtsbereich vorbereitet werden (Zahnimplantate).

Eine Verbindung mit einer zellulären Besiedlung in Bioreaktoren mit Stammzellen ist möglich.

Biologische Implantate (Gefäße, Herzklappen, Haut) und Membranen können ebenso mit EPO und oder GH und oder TPO beschichtet werden.

### 5. Behandlung der Osteoporose

EPO beschichtete Tricalziumphosphat Granula werden mittels einer Nadelpunktion in defizitäre / rarefizierte Wirbelkörper in einer autologen Plasmalösung eingebracht. Dort werden die Granula beschleunigt in endogenen Knochen umgebaut und der Degenerationsprozess in einen anabolen Effekt umgewandelt.

Der Effekt kann bei akut einbruchgefährdeten Wirbelkörper verwendet werden. Eine Kombination mit einem Besiedlungsprozess vorteilhafterweise mit Stammzellen aus dem autologen Knochenmark bzw. dem Periost und Blut und / oder Fettgewebe.

### 6. Indikation Knorpelregeneration

Knorpelzellen stellen stark bradytrophe Gewebe dar. Durch ein regionales Trauma und Abrasion entstehen Entzündungsprozesse, die in eine Arthitis münden können. Durch Gabe von EPO in den Gelenkspalt bzw. systemisch und / oder in Kombination mit zellulären Transplantaten von Chondrozyten bzw. osteochonralen Zylindern wird die Geweberegeneration und der strukturelle Neuaufbau gefördert. Die kombinierte systemische bzw. subkkutane Gabe von 10000 IE / Tag ist möglich.

### 7. Indikation Hauterkrankungen

Schlecht heilende Wunden werden nach Vorbereitung des Wundbettes erfindungsgemäß mit EPO oder TPO (Derivate, Analoga, Teile) beschichtet. Hierzu wird bevorzugt ein mechanisches Debridement durchgeführt. In die Blutkoagel werden 10 000 IE EPO eingebracht. Diese Prozess kann bis zur Reinigung des Wundgrundes wiederholt werden.

Das strukturelle Wachstum beginnt nach 2-3 Tagen führt zur beschleunigten Ausbildung eines Granulaitionsgewebes.

### 8. Indikation entzündliche Darmerkrankungen

Bei Entzündungsphänomenen des Darmtraktes mit Gewichtsverlust und Anämie hat sich herausgestellt dass die Anämie nicht ein Sekundärphänomen auf Grund der schlechten Nahrungsresorption ist, sondern Begleiterscheinung eines originären EPO Mangels sein kann. Die Gabe von EPO in 10 000 IE / Tag führt zu einer Verbesserung der Darmwiederherstellung / Regeneration.

### 9. Indikation Neuroregeneration

Nach Druchtrennungen des Rückenmarks führt EPO zu einem strukturellem Wachstum der Neurone und Neuaussprossung der Axone. Unterstützend wirkt die Gabe von Vitamin C.

EPO kann regional in Verbindung mit Fibrinkleber / autologem Plasma und / oder körpereigenen Stammzellen (Knochenmark, Blut CD 34, aus Fettzellen, Periost, Nabelschnur) gegeben werden.

### 10. Indikation Parkinson / Beispiel einer chronischen Erkrankung mit bereits abgeklungener Entzündungsreaktion

Autologe Makrophagen, die durch Stimulation mit degradablen Partikeln stimuliert wurden, werden stereotaktisch in degenerierte Areale integriert. Parallel hierzu wird EPO (10 000 IE) in Verbindung mit autologen Stammzellen (0.3 ml) in das Areal injeziert. Parallel hierzu wird EPO über 2 Wochen stemisch verabreicht. Dieses Stimulationsprinzip der Induzierung einer Entzündung durch Makrophagen kann auch bei anderen chronischen Erkrankungen eingesetzt werden, bei denen kein akutes Trauma oder eine akute Entzündungsreaktion vorherrscht.

## Patentansprüche

1. Verwendung von hämatopoietischen Wachstumsfaktoren, insbesondere von Erythropoietin (EPO) oder von Thrombopoietin (TPO), oder deren Derivate, Analoga oder Teile zur Herstellung eines Medikaments zur Förderung der strukturellen Regeneration von Gewebe.

2. Verwendung nach Anspruch 2, **gekennzeichnet durch** die Regeneration traumatisierten Gewebes.

3. Verwendung nach Anspruch 1 oder 2, **gekennzeichnet durch** die Verwendung der Rezeptor-Binde-Domäne der Wachstumsfaktoren bzw. deren Derivate oder Analoga.

4. Verwendung nach Anspruch 1 oder 2, **dadurch gekennzeichnet, dass** ein mimetisches Peptid eines der Wachstumsfaktoren, insbesondere EMP oder DMP, verwendet wird.

5. Verwendung nach einem der Ansprüche 1 bis 4, **dadurch gekennzeichnet, dass** die Wachstumsfaktoren oder deren Derivate, Analoga oder Teile gegenüber dem nativen Wachstumsfaktor zusätzliche Glykosylierungsstellen aufweisen.

6. Verwendung nach einem der Ansprüche 1 bis 5, **dadurch gekennzeichnet, dass** die Wachstumsfaktoren oder deren Derivate, Analoga oder Teile mit PEG konjugiert sind.

7. Verwendung nach Anspruch 1 oder 2, **gegenzeichnet durch** die Verwendung des *Novel Erythropoiesis Stimulating Protein* (NESP).

8. Verwendung eines EPO induzierenden Faktors zur Herstellung eines Medikaments zur Förderung der strukturellen Geweberegeneration.

9. Verwendung nach einem der Ansprüche 1 bis 8, **dadurch gekennzeichnet, dass** zusätzlich einer der folgenden Faktoren eingesetzt wird: Somatostatin, Leukemia Inhibitory Factor (LIF), "Ciliary Neurotropic Factor" (CNTF), "Transforming Growth Factor beta" (TGF beta), Prostaglandine, Granulozyten-Makrophagen-stimulierender Faktor (GM-CSF), Granulozyten-stimulierender Faktor (G-CSF), Growth Hormone Releasing Hormone (GHRH), Thyrotropin-releasing Homone (TRH), Gonadotropin-releasing Hormone (GnRH), Corticotropin-releasing Hormone (CRH), Dopamin, Antidiuretic Hormon (ADH), Oxytocin, Prolactin, Adrenocorticotropin, beta-Celltropin, Lutrotropin, Vasopressin, Nervenregenerationsfaktoren, vorzugsweise Nerve Growth Factor (NGF), Gefäßregenerationsfaktoren, vorzugsweise Vascular Endothelial Growth Factor (VEGF) oder Plateled Derived Growth Factor (PDGF).

10. Verwendung nach einem der Ansprüche 1 bis 9, **dadurch gekennzeichnet, dass** Endothelzellen anwesend sind.

11. Verwendung nach einem der Ansprüche 1 bis 10, **dadurch gekennzeichnet, dass** die Regeneration des Gewebes lokal gelenkt wird.

12. Verwendung nach einem der Ansprüche 1 bis 10, **dadurch gekennzeichnet, dass** das Medikament enthaltend einen oder mehrere der Faktoren nach einem der Ansprüche 1 bis 9 topisch verabreicht wird.

13. Verwendung nach einem der Ansprüche 1 bis 10, **dadurch gekennzeichnet, dass** das Medikament enthaltend eine oder mehrere der Faktoren nach einem der Ansprüche 1 bis 9 systemisch verabreicht wird.

14. Verwendung nach einem der Ansprüche 1 bis 13, **dadurch gekennzeichnet, dass** der Wachstumsprozess durch eine Trägerstruktur unterstützt wird.

15. Verwendung nach Anspruch 13, **dadurch gekennzeichnet, dass** die Trägerstruktur mit einem der Faktoren nach einem der Ansprüche 1 bis 9 behandelt wird.

16. Verwendung nach Anspruch 14 oder 15, **dadurch gekennzeichnet, dass** als Trägerstruktur ein Implantat, ein Transplantat oder ein Trägermaterial zum Wachstum von Zellen verwendet wird.

17. Verwendung nach einem der Ansprüche 14 bis 16, **dadurch gekennzeichnet, dass** die Trägerstruktur mit Zellen, vorzugsweise gewebespezifischen Zellen, Vorläuferzellen, Knochenmarkszellen, peripherem Blut, Fettgewebe oder Fasergewebe vorbesiedelt oder für die *in vivo* Besiedelung bzw. das induktive Remodelling *in vitro* vorbereitet wurde.

18. Verwendung nach Anspruch 17, **dadurch gekennzeichnet, dass** als Zellen adulte Progenitorzellen, gewebespezifische Zellen, vorzugsweise Osteoblasten, Fibroblasten, Hepatozyten oder glatte Muskelzellen eingesetzt werden.

19. Verwendung nach einem der Ansprüche 1 bis 18, **dadurch gekennzeichnet, dass** die sich während des Regenerationsprozesses bildenden Zellaggregate eingekapselt und gegebenenfalls eingefroren werden.

20. Verwendung nach einem der Ansprüche 1 bis 19, **gekennzeichnet durch** die Regeneration von Nerven-, Muskel-, Epithel- oder Bindegewebe sowie daraus abgeleiteter Organe und Strukturen.

21. Verwendung nach einem der Ansprüche 1 bis 20 zur Regeneration der Leber, insbesondere bei Leberzirrhose, Hepatitis, akutem oder chronischem Leberversagen.

22. Verwendung nach einem der Ansprüche 1 bis 20, **gekennzeichnet durch** die Behandlung zur Regeneration von Knochen, Knorpel, sowie Gewebe endokriner Organe, des Herzmuskels, von Herzklappen, Venenklappen, Arterienklappen, Haut, Gefäßen, Aorten, Sehnen, Comea, Tracea, Nerven, Miniskus, Diskus intervertebralis, Darmepithels, Ureteren, Urethra oder der Blase, sowie zur Behandlung von Degenerationserkrankungen oder zur Unterstützung der Geweberegeneration bei chronischen Entzündungen wie z.B. bei Morbus Crohn, Colitis Ulcerosa der diabetischen Ulzera, Gingiva oder zur Anregung der Gefäßneubildung im Anschluss an eine Gewebeverletzung.

23. Trägerstruktur enthaltend mindestens einen hämatopoietischen Wachstumsfaktor oder dessen Derivate, Analoga oder Teile nach einem der Ansprüche 1 bis 7 oder einen EPO induzierenden Faktor nach Anspruch 8.

24. Trägerstruktur nach Anspruch 23 enthaltend zusätzlich mindestens einen der folgenden Wachstumsfaktoren: Somatostatin, Leukemia Inhibitory Factor (LIF), "Ciliary Neurotropic Factor" (CNTF), "Transforming Growth Factor beta" (TGF beta), Prostaglandine, Granulozyten-Makrophagen-stimulierender Faktor (GM-CSF), Granulozyten-stimulierender Faktor (G-CSF), Growth Hormone Releasing Hormone (GHRH), Thyrotropin-releasing Homone (TRH), Gonadotropin-releasing Hormone (GnRH), Corticotropin-releasing Hormone (CRH), Dopamin, Antidiuretic Hormon (ADH), Oxytocin, Prolactin, Adrenocorticotropin, beta-Celltropin, Lutrotropin, Vasopressin, Nervenregenerationsfaktoren, vorzugsweise Nerve Growth Factor (NGF), Gefäßregenerationsfaktoren, vorzugsweise Vascular Endothelial Growth Factor (VEGF) oder Plateled Derived Growth Factor (PDGF).

25. Trägerstruktur nach Anspruch 23 oder 24, **dadurch gekennzeichnet, dass** die Trägerstruktur ein Implantat, ein Transplantat, ein Trägermaterial zum Wachstum von Zellen, ein Stent, ein Patch, ein Katheter, Haut, ein Hydrogel, ein Knochenersatzmaterial, ein allogenes, autologes oder xenogenes, azellularisiertes oder nicht-azellularisiertes Gewebe, ein synthetisches Gewebe, ein Feeder-Layer oder ein Fließ ist.

26. Trägerstruktur nach einem der Ansprüche 23 bis 25, **dadurch gekennzeichnet, dass** die Trägerstruktur mit Zellen, vorzugsweise gewebespezifischen Zellen, Vorläuferzellen, Knochenmarkszellen, peripherem Blut, Fettgewebe oder Fasergewebe vorbesiedelt ist.

27. Trägerstruktur nach einem der Ansprüche 23 bis 25, **dadurch gekennzeichnet, dass** die Wachstumfaktoren bzw. deren Derivate, Analoga oder Teile nach einem der Ansprüche 1 bis 9 in eine biodegradablen Polymerschicht eingebettet sind.

28. Verfahren zur Herstellung einer Trägerstruktur für die Zellregeneration, **dadurch gekennzeichnet, dass** die Trägerstruktur mit mindestens einem der Wachstumsfaktoren, dessen Derivate, Analoga oder Teile nach einem der Ansprüche 1 bis 9 beschichtet wird.

29. Verfahren nach Anspruch 28, **gekennzeichnet durch** die Aktivierung der Trägerstruktur, vorzugsweise **durch** Plasmaionisation oder Laserbestrahlung.

30. Verfahren nach mindestens einem der Ansprüche 28 und 29, **dadurch gekennzeichnet, dass** die Trägerstruktur *in vitro* mit Zellen, vorzugsweise gewebespezifischen Zellen, Vorläuferzellen, Knochenmarkszellen, peripherem Blut, Fettgewebe oder Fasergewebe vorbesiedelt wird.

31. Verwendung nach einem der Ansprüche 1 bis 22, **dadurch gekennzeichnet, dass** wenigstens einige der Verfahrensschritte zur Förderung der Geweberegeneration ganz oder teilweise *in vitro* durchgeführt werden.

32. Verwendung nach der Anspruch 31, **dadurch gekennzeichnet, dass** der Wachstumsprozess durch Stammzellengabe aus dem Knochenmark, Blut, Gewebe, Fettgewebe, Nabelschnurgewebe bzw. Blut unterstützt wird.
